# EUROPÄISCHE PATENTSCHRIFT

(11) **EP 3 571 181 B1**
(45) Veröffentlichungstag und Bekanntmachung des Hinweises auf die Patenterteilung: **24.06.2026**
(21) Anmeldenummer: 17797166.0
(22) Anmeldetag: 14.11.2017
(51) Int. Cl.: C07C 67/08, C07C 69/73, C07C 67/26, C07C 69/78, C07C 67/62, C07D 207/16

(54) **LAGERSTABILER GLYCERIN(METH)ACRYLATCARBONSÄUREESTER**
STORAGE-STABLE GLYCERIN (METH) ACRYLATE CARBOXYLIC ACID ESTERS
ESTER D'ACIDE CARBOXYLIQUE GLYCÉRINE(MÉTH)ACRYLATE STABLE AU STOCKAGE

(30) Priorität: 20.01.2017 EP 17152399
(43) Veröffentlichungstag der Anmeldung: 27.11.2019
(73) Patentinhaber: Evonik Operations GmbH, 45128 Essen (DE)
(72) Erfinder: HARTMANN, Patrik, 64572 Buettelborn (DE); KAUFMANN, Marita, 64347 Griesheim (DE); KRILL, Steffen, 64367 Mühltal (DE); SCHÜTZ, Thorben, 64665 Alsbach-Hähnlein (DE); TRESKOW, Marcel, 64293 Darmstadt (DE); WITTKOWSKI, Andrea, 64823 Gross-Umstadt (DE)
(74) Vertreter: Evonik Patent Association
(86) Internationale Anmeldenummer: PCT/EP2017/079124
(87) Internationale Veröffentlichungsnummer: WO 2018/133972

(56) Entgegenhaltungen:
- EP-A2- 0 054 700
- WO-A1-2015/124458

## Beschreibung

Die vorliegende Erfindung beschreibt lagerstabile Glycerin(meth)acrylatcarbonsäureester und ein Verfahren zur Herstellung dieser lagerstabilen Glycerin(meth)acrylatcarbonsäureester.

Verfahren zur Herstellung von Glycerin(meth)acrylatcarbonsäureestern sind aus dem Stand der Technik bekannt.

Glycerindimethacrylat wird aus Glycidylmethacrylat und Methacrylsäure in Anwesenheit katalytischer Mengen eines quaternären Ammoniumsalzes (im speziellen Fall ist dies Benzyltriethylammoniumchlorid) hergestellt.

Der Stand der Technik beschreibt Verfahren zur Herstellung von Glycerindimethacrylat aus Methacrylsäure und Glycidylmethacrylat in Gegenwart von Katalysatoren. Vorzugsweise werden quartäre Ammoniumsalze als Katalysatoren verwendet.

Bei dieser Reaktion verbleiben < 2 Prozent unumgesetztes Glycidylmethacrylat im Produktgemisch. Vom Glycidylmethacrylat ist bekannt, dass es in in vitro-Tests Genotoxizität aufweist (OECD SIDS Report "Glycidyl methacrylate", 2000).

In der EP 0054700 wird ein Verfahren zur Herstellung von Glycerindimethacrylat beschrieben. Dabei wird zu einer Mischung aus Methacrylsäure, Benzyltriethylammoniumchlorid und p-Methoxyphenol bei einer Temperatur von 80°C Glycidylmethacrylat gegeben. Nach der Aufarbeitung mit Natriumcarbonatlösung, Phasentrennung und Trocknung wird fraktioniert destilliert. Die Ausbeute beträgt nur 75%. Zudem polymerisiert das Produkt bei der destillativen Aufreinigung. Die Destillation kann daher nur mit kleinen Mengen erfolgen, was für eine großtechnische Umsetzung nicht wirtschaftlich ist.

Die WO2015/124458 offenbart ein Verfahren zur Herstellung von hochreinem Glycerindimethacrylat. Es wird die Verwendung eines sauren Adsorbens zur Nachbehandlung (Aufreinigung) eines glycidylmethacrylathaltigen Glycerindimethacrylats beschrieben, der gemäß EP 0054700 hergestellt wurde.

In der EP 1693359 wird die Herstellung von Hydroxyalkyl(meth)acrylaten beschrieben. Dabei werden epoxidgruppenhaltige Verbindungen mit Carbonsäuren in Gegenwart von Lewis Säuren umgesetzt, die jeweils mindestens eine unmittelbar gebundene Di(cyclo)alkylaminogruppe tragen. Die Beispiele zeigen, dass das Produkt im GPC zu maximal 80 Flächen-% gefunden wurde. Zudem wird ein zusätzlicher Verfahrensschritt beschrieben, der den Epoxidgehalt unter 0,2 Gew.-% bringen soll. Die verwendeten Lewis-Säuren sind für großtechnische Umsetzungen uninteressant, da sie auf dem Weltmarkt nur in Kleinstmengen zu hohen Preisen erhältlich sind.

In der CS 200395 wird ein Verfahren zur Herstellung einer Monomermischung beschrieben, durch Umsetzung von (Meth)acrylsäure mit Glycidyl(meth)acrylat in Anwesenheit von Hydrochinon und Triethylamin. Die anschließende mehrstufige Aufreinigung umfasst eine Extraktion mit einer wäßrigen Alkalimetallcarbonatlösung und anschließender Extraktion mit einer wäßrigen Schwefelsäure und abschließend eine Extraktion mit Wasser.

Die WO2005090281 beschreibt ein Dentalmaterial, welches eine (Meth)acrylsäure und Verbindungen mit Salicylsäure-Struktur enthält die mit di,- tri oder höher valenten Verbindungen vernetzt werden. Es wird 4-Hydroxysalicysäure mit Natriumhydroxid und Glycidylmethacrylat umgesetzt. Es entsteht das para-verknüpfte Additionsprodukt von 4-Hydroxysalicysäure und Glycidylmethacrylat mit lediglich 20% Ausbeute.

Die WO0059982 beschreibt ein Verfahren zur Herstellung von stark vernetzten Polyestern in dem Polycarboxylsäureanhydrid mit einem Polyol in Anwesenheit eines Amins zur Reaktion gebracht wird und anschließend die entstandene Säure mit Glycidyl(meth)acrylat oder Allylglycidylether umgesetzt wird. Dieses Produkt wird anschließend mit einem Anhydrid umgesetzt.

Die EP951896 beschreibt ein Verfahren zur Herstellung von zwei- und mehrkomponentigen, radikalisch polymerisierbaren Dentalwerkstoffen. Nach der Umsetzung von Acrylsäure, Mercaptoethanol und einem Radikalstarter wird die Hälfte der erhaltenen Oligocarbonsäure direkt mit Glycidylmethacrylat weiter umgesetzt. Auf Grund des großen Säureüberschusses erfolgt keine Disproportionierung.

Bei der Lagerung von Glycerindimethacrylat kann es zur Disproportionierung des Produktes kommen. Dabei entstehen Glycerinmonomethacrylat und Glycerintrimethacrylat.

Aufgabe war es, lagerstabile Glycerin(meth)acrylatcarbonsäureester und ein Verfahren zur Herstellung lagerstabiler Glycerin(meth)acrylatcarbonsäureester zur Verfügung zu stellen.

Die Aufgabe wurde gelöst durch lagerstabile Glycerin(meth)acrylatcarbonsäureester der Formel (I) 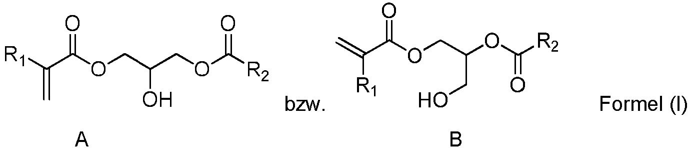 mit
R₁ = H oder CH₃
R₂=Wasserstoff, oder aliphatische Kohlenstoffe mit C1 bis C30, oder aliphatisch cyclische Kohlenstoffverbindungen mit einer Ringgröße von C4 bis C8, unsubstituiert oder substituiert, mit N, S, O oder P, oder halogenierte aliphatische Kohlenstoffverbindungen mit C1 bis C8, oder aromatische Kohlenstoffverbindungen, oder heteroaromatische Kohlenstoffverbindungen mit N, S, O oder P substituiert, oder
ungesättigten aliphatischen Kohlenstoffverbindungen mit C2 bis C30,
wobei systemeigene Carbonsäuren der Formel (II) 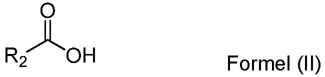 wobei
   R₂=Wasserstoff, oder aliphatische Kohlenstoffe mit C1 bis C30, oder aliphatisch cyclische Kohlenstoffverbindungen mit einer Ringgröße von C4 bis C8, unsubstituiert oder substituiert, mit N, S, O oder P, oder halogenierte aliphatische Kohlenstoffverbindungen mit C1 bis C8, oder aromatische Kohlenstoffverbindungen, oder heteroaromatische Kohlenstoffverbindungen mit N, S, O oder P substituiert, oder
   ungesättigten aliphatischen Kohlenstoffverbindungen mit C2 bis C30
   und systemfremde Carbonsäuren der Formel (III) 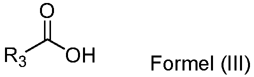 wobei
      R₃=Wasserstoff, oder aliphatische Kohlenstoffe mit C1 bis C30, oder aliphatisch cyclische Kohlenstoffverbindungen mit einer Ringgröße von C4 bis C8, unsubstituiert oder substituiert, mit N, S, O oder P, oder halogenierte aliphatische Kohlenstoffverbindungen mit C1 bis C8, oder aromatische Kohlenstoffverbindungen, oder heteroaromatische Kohlenstoffverbindungen mit N, S, O oder P substituiert, oder
   ungesättigten aliphatischen Kohlenstoffverbindungen mit C2 bis C30 einzeln oder in Mischungen und gegebenenfalls beliebige weitere systemfremde Broenstedsäuren in Summe im molaren Überschuss zum enthaltenden Glycidyl(meth)acrylat enthalten sind.

Der Begriff Säuren umfasst erfindungsgemäß sowohl systemeigene Carbonsäuren der Formel (II), als auch systemfremde Carbonsäuren der Formel (III) und deren Mischungen, aber auch systemfremde Broenstedsäuren. Systemeigene Carbonsäuren (II) umfasst Carbonsäuren, die mit Glycidyl(meth)acrylat umgesetzt zu den Glycerin(meth)acrylatcarbonsäureestern (I) reagieren.

Der molare Überschuss der Summe der Säuren im lagerstabilen Glycerin(meth)acrylatcarbonsäureester umfasst sowohl das Vorhandensein der systemeigenen Carbonsäuren (II) allein im Überschuss, als auch beliebige Säuregemische. Beispielsweise kann eine Carbonsäure, oder Mischungen verschiedener Carbonsäuren gemäß Formel (III) in Summe im molaren Überschuss enthalten sein. Eine weitere Alternative bieten jegliche Broenstedsäuren allein oder in Mischungen, aber auch in Mischungen mit systemeigenen Carbonsäuren der Formel (II) und/oder Carbonsäuren der Formel (III) und deren Mischungen.

Der molare Überschuss der Säuren in Summe liegt erfindungsgemäß bei einem molaren Verhältnis der Säuren zum enthaltenden Glycidyl(meth)acrylat von 1,001 zu 1 bis 5 zu 1.

Bevorzugt sind lagerstabile Glycerin(meth)acrylatcarbonsäureester mit einem molaren Verhältnis von Säure zu Glycidyl(meth)acrylat im Verhältnis von 1,01 zu 1 bis 2 zu 1, besonders bevorzugt im Verhältnis von 1,02 zu 1 bis 1,5 zu 1.

Das erfindungsgemäße Verhältnis Säure zu Glycidyl(meth)acrylat liegt vorzugsweise am Ende der Zugabe der Edukte systemeigene Carbonsäure (II) und Glycidyl(meth)acrylat vor.

Alternativ liegt dieses Verhältnis auch nach der Synthese, also vor der Lagerung vor.

Es hat sich gezeigt, dass vor der Lagerung die Einstellung des Verhältnisses durch die Zugabe sowohl der systemeignen Carbonsäure gemäß Formel (II), als auch mit systemfremden Carbonäuren der Formel (III) oder anorganischen, systemfremden Broenstedsäuren erfolgen kann. Besonders bevorzugt ist ein lagerstabiler Glycerin(meth)acrylatcarbonsäureester, dadurch gekennzeichnet, dass die systemeigene Carbonsäure eine (Meth)acrylsäure ist, mit einem molaren Verhältnis von (Meth)acrylsäure zu Glycidyl(meth)acrylat von 1,01 zu 1 bis 2 zu 1.

Die Aufgabe wurde auch durch die Bereitstellung eines Verfahrens zur Herstellung von lagerstabilem Glycerin(meth)acrylatcarbonsäureester gelöst. Das erfindungsgemäße Verfahren ist dadurch gekennzeichnet, dass systemeigene Carbonsäuren der Formel (II) 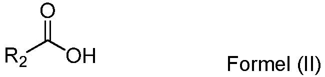 wobei
R₂=Wasserstoff, oder aliphatische Kohlenstoffe mit C1 bis C30, oder aliphatisch cyclische Kohlenstoffverbindungen mit einer Ringgröße von C4 bis C8, unsubstituiert oder substituiert, mit N, S, O oder P, oder halogenierte aliphatische Kohlenstoffverbindungen mit C1 bis C8, oder aromatische Kohlenstoffverbindungen, oder heteroaromatische Kohlenstoffverbindungen mit N, S, O oder P substituiert, oder
ungesättigten aliphatischen Kohlenstoffverbindungen mit C2 bis C30
und Glycidyl(meth)acrylat in Anwesenheit eines Katalysators bei Temperaturen von 20-130°C umgesetzt werden, indem systemeigenen Carbonsäure (II) vorgelegt und Glycidyl(meth)acrylat kontinuierlich zugegeben wird, und gegebenenfalls systemfremde Carbonsäuren der Formel (III) 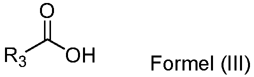 wobei
   R₃=Wasserstoff, oder aliphatische Kohlenstoffe mit C1 bis C30, oder aliphatisch cyclische Kohlenstoffverbindungen mit einer Ringgröße von C4 bis C8, unsubstituiert oder substituiert, mit N, S, O oder P, oder halogenierte aliphatische Kohlenstoffverbindungen mit C1 bis C8, oder aromatische Kohlenstoffverbindungen, oder heteroaromatische Kohlenstoffverbindungen mit N, S, O oder P substituiert, oder
   ungesättigten aliphatischen Kohlenstoffverbindungen mit C2 bis C30 einzeln oder in Mischungen
und/oder gegebenenfalls beliebige weitere systemfremde Broenstedsäuren in Summe im molaren Überschuss zum enthaltenden Glycidyl(meth)acrylat zugegeben werden.

Es wurde gefunden, dass durch gezielte Einstellung des Verhältnisses der Restgehalte von Säure und Glycidyl(meth)acrylat im Endprodukt eine deutlich verbesserte Lagerstabilität durch Unterbindung der Disproportionierung erreicht werden kann.

Lagerstabil ist ein Produkt für die Zeit in welches es nach Aufbewahrung bei 30°C oder höher noch die vorgegebene Produktspezifikation einhält. Definiert wird, dass bei der Lagerung bei vorgegebenen Temperaturen, beispielsweise bei 30 °C oder 50°C, der Gehalt an Disproportionierungsprodukten einen Maximalwert nicht überschreitet. Gleichzeitig darf ein Minimalwert für Diester nicht unterschritten werden. Definiert werden Lagerzeiten von 1 Monat, 3 Monaten, 6 Monaten und 8 Monaten.

Glycerin(meth)acrylatcarbonsäureester sind erfindungsgemäß lagerstabil, wenn die Grenzwerte für Diester von 85 Gew.-% nicht unter- bzw. für Triester von 3 Gew.-% nicht überschritten werden. Überraschend wurde gefunden, dass bei einem Säureüberschuss die Glycerin(meth)acrylatcarbonsäureester bei 30°C immer über 8 Monate lagerstabil sind. Erfolgt die Lagerung bei 50°C kann eine Lagerstabilität von mindestens 3 Monaten, bevorzugt von über 6 Monaten, ganz besonders bevorzugt über 8 Monate erreicht werden.

Die Einstellung des molaren Verhältnisses von Säure zu Glycidyl(meth)acrylat im Verhältnis von 1,001 zu 1 bis 5 zu 1 kann durch den Einsatz entsprechender Mengen systemeigener Carbonsäuren erfolgen. Bevorzugt wird das molaren Verhältnisses von Carbonsäure zu Glycidyl(meth)acrylat im Verhältnis von 1,01 zu 1 bis 2 zu 1, besonders bevorzugt von 1,02 zu 1 bis 1,5 zu 1, eingestellt. Diese molaren Verhältnisse liegen erfindungsgemäß am Ende der Zugabe der Edukte vor.

Überraschend wurde gefunden, dass unabhängig vom molaren Verhältnis der Carbonsäure zu Glycidyl(meth)acrylat am Ende der Zugabe der Edukte ein lagerstabiler Glycerin(meth)acrylatcarbonsäureester hergestellt werden kann, indem nach der Synthese, insbesondere vor der Lagerung durch Zugabe von Säuren ein molares Verhältnis von 1,001 zu 1 bis 5 zu 1 eingestellt wird. Bevorzugt wird das molare Verhältnis von Säure zu Glycidyl(meth)acrylat von 1,01 zu 1 bis 2 zu 1, besonders bevorzugt von 1,02 zu 1 bis 1,5 zu 1, vor der Lagerung ggf. durch Zugabe einer Säure eingestellt.

Es wurde gefunden, dass systemfremde Carbonsäuren geeignet sind, aber auch systemfremde Broenstedsäuren zugegeben werden können, um das erforderliche molare Verhältnis von Säure zu Glycidyl(meth)acrylat einzustellen.

Der molare Gehalt von Säure im Endprodukt muss bei der Lagerung größer sein als der molare Gehalt an Glycidyl(meth)acrylat.

Der Glycerin(meth)acrylatcarbonsäureester liegt als Isomerengemisch vor. Das Isomerenverhältnis ist abhängig von den Reaktionsbedingungen. Die Epoxidringöffnung verschiebt sich in Abhängigkeit von der Reaktiontemperatur und beeinflusst somit die Verteilung der Isomere A und B aus Formel (I).

Als Edukte werden systemeigene Carbonsäuren der Formel (II) eingesetzt. 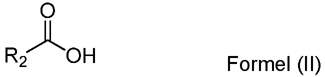 mit
R₁ = H oder CH₃
R₂=Wasserstoff, oder aliphatische Kohlenstoffe mit C1 bis C30, oder aliphatisch cyclische Kohlenstoffverbindungen mit einer Ringgröße von C4 bis C8, unsubstituiert oder substituiert, mit N, S, O oder P, oder halogenierte aliphatische Kohlenstoffverbindungen mit C1 bis C8, oder aromatische Kohlenstoffverbindungen, oder heteroaromatische Kohlenstoffverbindungen mit N, S, O oder P substituiert, oder ungesättigten aliphatischen Kohlenstoffverbindungen mit C2 bis C30. Die Carbonsäuren der Formel (II) umfassen auch funktionalisierte aliphatische Carbonsäuren mit funktionellen Gruppen ausgewählt aus der Gruppe der Hydroxide, Nitrile, Ester, Amide, Ketone, Thiole und Ether.

Geeignet sind Carbonsäuren aus folgenden Gruppen: Hydroxycarbonsäuren, gesättigte Carbonsäuren, ungesättigte und mehrfach ungesättigte Carbonsäuren, aromatische Carbonsäuren, cyclische und heterocyclische Carbonsäuren, heteroaromatische Carbonsäuren, sowie halogenierte Carbonsäuren.

Besonders bevorzugt sind die Carbonsäuren ausgewählt aus der Gruppe der Hydroxyisobuttersäure (HIBS), Methacrylsäure, Acrylsäure, Essigsäure, Buttersäure, Stearinsäure, Benzoesäure, Salicylsäure, Nicotinsäure, Prolin, Ölsäure, Milchsäure und Trichloressigsäure,

Die Carbonsäuren werden mit Glycidyl(meth)acrylat umgesetzt. Diese Schreibweise bedeutet sowohl Glycidylmethacrylat als auch Gycidylacrylat.

Das Verfahren wird bei Temperaturen zwischen 20 und 130°C, bevorzugt bei Temperaturen zwischen 85 und 110°C, durchgeführt.

Bei Temperaturen über 130°C besteht die akute Gefahr der Polymerisierung. Daher wird vorzugsweise die Carbonsäure vorgelegt und das Glycidyl(meth)acrylat so zudosiert, dass die Reaktionstemperatur im vorgegebenen Rahmen bleibt.

Carbonsäuren mit Schmelzpunkten oberhalb der Reaktionstemperatur erfordern den Einsatz von hochsiedenden, unter den Reaktionsbedingungen inerten Lösungsmitteln. Geeignete Lösungsmittel sind Toluol, Dimethylformamid, Nitrobenzol, Dibutylether, Chlobenzol und weitere Lösungsmittel aus der Gruppe der hochsiedenden Lösungsmittel.

Die Umsetzung erfolgt bevorzugt in Anwesenheit eines Katalysators.

Geeignete Katalysatoren sind quaternäre Alkylammoniumhalogenide, Triphenylphosphin, Triphenylphosphinoxid, Hexamethylentetramin, Tetramethylammoniumbromid, Tetrabutylammoniumbromid, N,N-Dimethylbenzylamin and aktive Cr(III)-Komplexe.

Besonders bevorzugt werden Benzyltriethylammoniumchlorid, Benzyltriethylammoniumbromid, Tetrabutylammoniumchlorid oder Tetrabutylammoniumbromid eingesetzt.

### Beispiele

In einem 1l-Witt'schen Topf mit Ölumlauf, Bodenablassventil, Porzellanblattrührer mit Rührmotor, 500ml Zulauftrichter sowie Thermometer und Lufteinleitung wurden die Carbonsäure, 0,05 g Hydrochinonmonomethylether und 9,60 g Benzyltriethylammoniumchlorid als Katalysator vorgelegt und auf 90°C erwärmt. Bei 90-91°C werden 300g Glycidyl(meth)acrylat innerhalb von 60min zugegeben. Nach Zulaufende wird der Ansatz auf 97°C erwärmt, die Temperatur steigt hierbei kurzzeitig auf max.100°C. Der Ansatz wird 10h bei 97°C gehalten, anschließend abgekühlt und abgelassen. Von dem so erhaltenen Produkt wurden Lagerstabilitätstest bei 30 und 50°C durchgeführt um die Neigung des Produktes zur Disproportionierung festzustellen.

### Herstellungsbeispiel 1 (nicht erfindungsgemäß)

Verfahren dadurch gekennzeichnet, dass als Carbonsäure 179 g Methacrylsäure verwendet wird. Molare Stöchiometrie (Glycidylmethacrylat: Carbonsäure) 1:0,985

| **Lagerstabilität bei 30°C** | | **Start** | **1 Monat** | **3 Monate** | **6 Monate** | **8 Monate** |
|---|---|---|---|---|---|---|
| **Diestergehalt [%]** | min. 85,00 | 86,1 % | 81,7 % | 53,0 % | 48,7 % | 48,4 % |
| **Triestergehalt [%]** | max. 3,00 | 1,6 % | 5,4 % | 28,1 % | 33,3 % | 33,7 % |
| **Methacrylsäure [%]** | | 0,40 % | n.b. | n.b. | n.b. | n.b. |
| **Glycidylmethacrylat [%]** | | 0,78 % | n.b. | n.b. | n.b. | n.b. |

| **Lagerstabilität bei 50°C** | | **Start** | **1 Monat** | **3 Monate** | **6 Monate** | **8 Monate** |
|---|---|---|---|---|---|---|
| **Diestergehalt [%]** | min. 85,00 | 86,1 % | 49,0 % | 46,8 % | 47,2 % | 47,0 % |
| **Triestergehalt [%]** | max. 3,00 | 1,6 % | 31,9 % | 33,6 % | 33,8 % | 33,6 % |
| **Methacrylsäure [%]** | | 0,40 % | n.b. | n.b. | n.b. | n.b. |
| **Glycidylmethacrylat [%]** | | 0,78 % | n.b. | n.b. | n.b. | n.b. |

| | | | | | | |
|---|---|---|---|---|---|---|
| Fazit: Das zum Ende der Zugabe der Edukte mit einer molaren Stöchiometrie von 1:0,985 hergestellte Produkt erwies sich als NICHT lagerstabil! | | | | | | |

### Herstellungsbeispiel 2 (nicht erfindungsgemäß)

### Molare Stöchiometrie (Glycidylmethacrylat:Carbonsäure) 1:1

Verfahren dadurch gekennzeichnet, dass die Durchführung analog Beispiel 1 durchgeführt wird, jedoch als Carbonsäure 181,7 g Methacrylsäure verwendet wird.

| **Lagerstabilität bei 30°C** | | **Start** | **1 Monat** | **3 Monate** | **6 Monate** | **8 Monate** |
|---|---|---|---|---|---|---|
| **Diestergehalt [%]** | min. 85,00 | 86,2 % | 81,8 % | 53,1 % | 48,8 % | 48,5 % |
| **Triestergehalt [%]** | max. 3,00 | 1,8 % | 6,1 % | 31,5 % | 37,4 % | 37,8 % |
| **Methacrylsäure [%]** | | 0,42 % | n.b. | n.b. | n.b. | n.b. |
| **Glycidylmethacrylat [%]** | | 0,72 % | n.b. | n.b. | n.b. | n.b. |

| **Lagerstabilität bei 50°C** | | **Start** | **1 Monat** | **3 Monate** | **6 Monate** | **8 Monate** |
|---|---|---|---|---|---|---|
| **Diestergehalt [%]** | min. 85,00 | 86,2 % | 49,1 % | 46,9 % | 47,2 % | 47,1 % |
| **Triestergehalt [%]** | max. 3,00 | 1,8 % | 35,8 % | 37,7 % | 37,9 % | 37,6 % |
| **Methacrylsäure [%]** | | 0,42 % | n.b. | n.b. | n.b. | n.b. |
| **Glycidylmethacrylat [%]** | | 0,72 % | n.b. | n.b. | n.b. | n.b. |

| | | | | | | |
|---|---|---|---|---|---|---|
| Fazit: Das zum Ende der Zugabe der Edukte mit einer molaren Stöchiometrie von 1:1 hergestellte Produkt erwies sich als NICHT lagerstabil! | | | | | | |

### Erfindungsgemäßes Beispiel 3

### Molare Stöchiometrie (Glycidylmethacrylat:Carbonsäure) 1:1,02

Verfahren dadurch gekennzeichnet, dass die Durchführung analog Beispiel 1 durchgeführt wird, jedoch als Carbonsäure 185,3 g Methacrylsäure verwendet wird.

| **Lagerstabilität bei 30°C** | | **Start** | **1 Monat** | **3 Monate** | **6 Monate** | **8 Monate** |
|---|---|---|---|---|---|---|
| **Diestergehalt [%]** | min. 85,00 | 89,3 % | 90,0 % | 89,8 % | 89,9 % | 89,3 % |
| **Triestergehalt [%]** | max. 3,00 | 1,5 % | 1,5 % | 1,5 % | 1,6 % | 1,9 % |
| **Methacrylsäure [%]** | | 0,45 % | n.b. | n.b. | n.b. | n.b. |
| **Glycidylmethacrylat [%]** | | 0,65 % | n.b. | n.b. | n.b. | n.b. |

| **Lagerstabilität bei 50°C** | | **Start** | **1 Monat** | **3 Monate** | **6 Monate** | **8 Monate** |
|---|---|---|---|---|---|---|
| **Diestergehalt [%]** | min. 85,00 | 89,3 % | 89,4 % | 89,3 % | 88,7 % | 87,2 % |
| **Triestergehalt [%]** | max. 3,00 | 1,5 % | 1,7 % | 2,0 % | 2,6 % | 3,0 % |
| **Methacrylsäure [%]** | | 0,45 % | n.b. | n.b. | n.b. | n.b. |
| **Glycidylmethacrylat [%]** | | 0,65 % | n.b. | n.b. | n.b. | n.b. |

| | | | | | | |
|---|---|---|---|---|---|---|
| Fazit: Das zum Ende der Zugabe der Edukte mit einer molaren Stöchiometrie von 1:1,02 hergestellte Produkt hatte eine Lagerstabilität von über 8 Monaten. | | | | | | |

### Herstellungsbeispiel 4 (nicht erfindungsgemäß)

Verfahren dadurch gekennzeichnet, dass die Durchführung analog Beispiel 1 durchgeführt wird, jedoch als Carbonsäure 124,8 g Essigsäure verwendet wird.

### Molare Stöchiometrie (Glycidylmethacrylat : Carbonsäure) 1:0,985

| **Lagerstabilität bei 30°C** | | **Start** | **1 Monat** | **3 Monate** | **6 Monate** | **8 Monate** |
|---|---|---|---|---|---|---|
| **Diestergehalt [%]** | min. 85,00 | 86,8 % | 82,4 % | 53,5 % | 49,1 % | 48,8 % |
| **Triestergehalt [%]** | max. 3,00 | 1,7 % | 5,8 % | 29,7 % | 35,3 % | 35,7 % |
| **Essigsäure [%]** | | 0,31 % | n.b. | n.b. | n.b. | n.b. |
| **Glycidylmethacrylat [%]** | | 0,88 % | n.b. | n.b. | n.b. | n.b. |

| **Lagerstabilität bei 50°C** | | **Start** | **1 Monat** | **3 Monate** | **6 Monate** | **8 Monate** |
|---|---|---|---|---|---|---|
| **Diestergehalt [%]** | min. 85,00 | 86,8 % | 49,5 % | 47,3 % | 47,6 % | 47,4 % |
| **Triestergehalt [%]** | max. 3,00 | 1,7 % | 33,8 % | 35,6 % | 35,8 % | 35,5 % |
| **Essigsäure [%]** | | 0,31 % | n.b. | n.b. | n.b. | n.b. |
| **Glycidylmethacrylat [%]** | | 0,88 % | n.b. | n.b. | n.b. | n.b. |

| | | | | | | |
|---|---|---|---|---|---|---|
| Fazit: Das zum Ende der Zugabe der Edukte mit einer molaren Stöchiometrie von 1:0,985 hergestellte Produkt erwies sich als NICHT lagerstabil! | | | | | | |

### Herstellungsbeispiel 5 (nicht erfindungsgemäß)

### Molare Stöchiometrie (Glycidylmethacrylat : Carbonsäure) 1:1

Verfahren dadurch gekennzeichnet, dass die Durchführung analog Beispiel 1 durchgeführt wird, jedoch als Carbonsäure 126,7 g Essigsäure verwendet wird.

| **Lagerstabilität bei 30°C** | | **Start** | **1 Monat** | **3 Monate** | **6 Monate** | **8 Monate** |
|---|---|---|---|---|---|---|
| **Diestergehalt [%]** | min. 85,00 | 87,0 % | 82,5 % | 53,6 % | 49,2 % | 48,9 % |
| **Triestergehalt [%]** | max. 3,00 | 1,8 % | 6,1 % | 31,3 % | 37,2 % | 37,6 % |
| **Essigsäure [%]** | | 0,33 % | n.b. | n.b. | n.b. | n.b. |
| **Glycidylmethacrylat [%]** | | 0,81 % | n.b. | n.b. | n.b. | n.b. |

| **Lagerstabilität bei 50°C** | | **Start** | **1 Monat** | **3 Monate** | **6 Monate** | **8 Monate** |
|---|---|---|---|---|---|---|
| **Diestergehalt [%]** | min. 85,00 | 87,0 % | 49,6 % | 47,3 % | 47,7 % | 47,5 % |
| **Triestergehalt [%]** | max. 3,00 | 1,8 % | 35,7 % | 37,5 % | 37,7 % | 37,5 % |
| **Essigsäure [%]** | | 0,33 % | n.b. | n.b. | n.b. | n.b. |
| **Glycidylmethacrylat [%]** | | 0,81 % | n.b. | n.b. | n.b. | n.b. |

| | | | | | | |
|---|---|---|---|---|---|---|
| Fazit: Das zum Ende der Zugabe der Edukte mit einer molaren Stöchiometrie von 1:1 hergestellte Produkt erwies sich als NICHT lagerstabil! | | | | | | |

### Erfindungsgemäßes Beispiel 6

### Molare Stöchiometrie (Glycidylmethacrylat : Carbonsäure) 1:1,02

Verfahren dadurch gekennzeichnet, dass die Durchführung analog Beispiel 1 durchgeführt wird, jedoch als Carbonsäure 129,3 g Essigsäure verwendet wird.

| **Lagerstabilität bei 30°C** | | **Start** | **1 Monat** | **3 Monate** | **6 Monate** | **8 Monate** |
|---|---|---|---|---|---|---|
| **Diestergehalt [%]** | min. 85,00 | 89,4 % | 90,1 % | 89,9 % | 90,0 % | 89,3 % |
| **Triestergehalt [%]** | max. 3,00 | 1,7 % | 1,8 % | 1,7 % | 1,9 % | 2,2 % |
| **Essigsäure [%]** | | 0,35 % | n.b. | n.b. | n.b. | n.b. |
| **Glycidylmethacrylat [%]** | | 0,73 % | n.b. | n.b. | n.b. | n.b. |

| **Lagerstabilität bei 50°C** | | **Start** | **1 Monat** | **3 Monate** | **6 Monate** | **8 Monate** |
|---|---|---|---|---|---|---|
| **Diestergehalt [%]** | min. 85,00 | 89,4 % | 89,5 % | 89,4 % | 88,8 % | 87,3 % |
| **Triestergehalt [%]** | max. 3,00 | 1,7 % | 2,1 % | 2,4 % | 3,0 % | 3,5 % |
| **Essigsäure [%]** | | 0,35 % | n.b. | n.b. | n.b. | n.b. |
| **Glycidylmethacrylat [%]** | | 0,73 % | n.b. | n.b. | n.b. | n.b. |

| | | | | | | |
|---|---|---|---|---|---|---|
| Fazit: Das zum Ende der Zugabe der Edukte mit einer molaren Stöchiometrie von 1:1,02 hergestellte Produkt hatte eine Lagerstabilität von über 6 Monaten. | | | | | | |

### Herstellungsbeispiel 7 (nicht erfindungsgemäß)

Verfahren dadurch gekennzeichnet, dass die Durchführung analog Beispiel 1 durchgeführt wird, jedoch als Carbonsäure 183,2 g Buttersäure verwendet wird.

### Molare Stöchiometrie (Glycidylmethacrylat : Carbonsäure 1:0,985

| **Lagerstabilität bei 30°C** | | **Start** | **1 Monat** | **3 Monate** | **6 Monate** | **8 Monate** |
|---|---|---|---|---|---|---|
| **Diestergehalt [%]** | min. 85,00 | 86,0 % | 81,6 % | 53,0 % | 48,7 % | 48,4 % |
| **Triestergehalt [%]** | max. 3,00 | 1,8 % | 6,2 % | 31,9 % | 37,9 % | 38,3 % |
| **Buttersäure [%]** | | 0,40 % | n.b. | n.b. | n.b. | n.b. |
| **Glycidylmethacrylat [%]** | | 0,78 % | n.b. | n.b. | n.b. | n.b. |

| **Lagerstabilität bei 50°C** | | **Start** | **1 Monat** | **3 Monate** | **6 Monate** | **8 Monate** |
|---|---|---|---|---|---|---|
| **Diestergehalt [%]** | min. 85,00 | 86,0 % | 49,0 % | 46,8 % | 47,1 % | 47,0 % |
| **Triestergehalt [%]** | max. 3,00 | 1,8 % | 36,3 % | 38,2 % | 38,4 % | 38,1 % |
| **Buttersäure [%]** | | 0,40 % | n.b. | n.b. | n.b. | n.b. |
| **Glycidylmethacrylat [%]** | | 0,78 % | n.b. | n.b. | n.b. | n.b. |

| | | | | | | |
|---|---|---|---|---|---|---|
| Fazit: Das zum Ende der Zugabe der Edukte mit einer molaren Stöchiometrie von 1:0,985 hergestellte Produkt erwies sich als NICHT lagerstabil! | | | | | | |

### Herstellungsbeispiel 8 (nicht erfindungsgemäß)

### Molare Stöchiometrie (Glycidylmethacrylat : Carbonsäure) 1:1

Verfahren dadurch gekennzeichnet, dass die Durchführung analog Beispiel 1 durchgeführt wird, jedoch als Carbonsäure 186 g Buttersäure verwendet wird.

| **Lagerstabilität bei 30°C** | | **Start** | **1 Monat** | **3 Monate** | **6 Monate** | **8 Monate** |
|---|---|---|---|---|---|---|
| **Diestergehalt [%]** | min. 85,00 | 87,0 % | 82,6 % | 53,6 % | 49,3 % | 49,0 % |
| **Triestergehalt [%]** | max. 3,00 | 1,9 % | 6,4 % | 33,2 % | 39,4 % | 39,8 % |
| **Buttersäure [%]** | | 0,42 % | n.b. | n.b. | n.b. | n.b. |
| **Glycidylmethacrylat [%]** | | 0,71 % | n.b. | n.b. | n.b. | n.b. |

| **Lagerstabilität bei 50°C** | | **Start** | **1 Monat** | **3 Monate** | **6 Monate** | **8 Monate** |
|---|---|---|---|---|---|---|
| **Diestergehalt [%]** | min. 85,00 | 87,0 % | 49,6 % | 47,4 % | 47,7 % | 47,5 % |
| **Triestergehalt [%]** | max. 3,00 | 1,9 % | 37,8 % | 39,7 % | 39,9 % | 39,7 % |
| **Buttersäure [%]** | | 0,42 % | n.b. | n.b. | n.b. | n.b. |
| **Glycidylmethacrylat [%]** | | 0,71 % | n.b. | n.b. | n.b. | n.b. |

| | | | | | | |
|---|---|---|---|---|---|---|
| Fazit: Das zum Ende der Zugabe der Edukte mit einer molaren Stöchiometrie von 1:1 hergestellte Produkt erwies sich als NICHT lagerstabil! | | | | | | |

### Erfindungsgemäßes Beispiel 9

### Molare Stöchiometrie (Glycidylmethacrylat : Carbonsäure) 1:1,02

Verfahren dadurch gekennzeichnet, dass die Durchführung analog Beispiel 1 durchgeführt wird, jedoch als Carbonsäure 189,7 g Buttersäure verwendet wird.

| **Lagerstabilität bei 30°C** | | **Start** | **1 Monat** | **3 Monate** | **6 Monate** | **8 Monate** |
|---|---|---|---|---|---|---|
| **Diestergehalt [%]** | min. 85,00 | 88,9 % | 89,6 % | 89,4 % | 89,5 % | 88,9 % |
| **Triestergehalt [%]** | max. 3,00 | 1,7 % | 1,8 % | 1,7 % | 1,9 % | 2,2 % |
| **Buttersäure [%]** | | 0,46 % | n.b. | n.b. | n.b. | n.b. |
| **Glycidylmethacrylat [%]** | | 0,64 % | n.b. | n.b. | n.b. | n.b. |

| **Lagerstabilität bei 50°C** | | **Start** | **1 Monat** | **3 Monate** | **6 Monate** | **8 Monate** |
|---|---|---|---|---|---|---|
| **Diestergehalt [%]** | min. 85,00 | 88,9 % | 89,1 % | 88,9 % | 88,4 % | 86,9 % |
| **Triestergehalt [%]** | max. 3,00 | 1,7 % | 2,0 % | 2,4 % | 3,0 % | 3,5 % |
| **Buttersäure [%]** | | 0,46 % | n.b. | n.b. | n.b. | n.b. |
| **Glycidylmethacrylat [%]** | | 0,64 % | n.b. | n.b. | n.b. | n.b. |

| | | | | | | |
|---|---|---|---|---|---|---|
| Fazit: Das zum Ende der Zugabe der Edukte mit einer molaren Stöchiometrie von 1:1,02 hergestellte Produkt hatte eine Lagerstabilität von über 6 Monaten. | | | | | | |

### Herstellungsbeispiel 10 (nicht erfindungsgemäß)

Verfahren dadurch gekennzeichnet, dass die Durchführung analog Beispiel 1 durchgeführt wird, jedoch als Carbonsäure 591,4 g Stearinsäure verwendet wird.

### Molare Stöchiometrie (Glycidylmethacrylat : Carbonsäure) 1:0,985

| **Lagerstabilität bei 30°C** | | **Start** | **1 Monat** | **3 Monate** | **6 Monate** | **8 Monate** |
|---|---|---|---|---|---|---|
| **Diestergehalt [%]** | min. 85,00 | 87,0 % | 82,5 % | 53,5 % | 49,2 % | 48,9 % |
| **Triestergehalt [%]** | max. 3,00 | 1,3 % | 4,2 % | 21,7 % | 25,8 % | 26,1 % |
| **Stearinsäure [%]** | | 0,71 % | n.b. | n.b. | n.b. | n.b. |
| **Glycidylmethacrylat [%]** | | 0,42 % | n.b. | n.b. | n.b. | n.b. |

| **Lagerstabilität bei 50°C** | | **Start** | **1 Monat** | **3 Monate** | **6 Monate** | **8 Monate** |
|---|---|---|---|---|---|---|
| **Diestergehalt [%]** | min. 85,00 | 87,0 % | 49,6 % | 47,3 % | 47,7 % | 47,5 % |
| **Triestergehalt [%]** | max. 3,00 | 1,3 % | 24,7 % | 26,0 % | 26,2 % | 26,0 % |
| **Stearinsäure [%]** | | 0,71 % | n.b. | n.b. | n.b. | n.b. |
| **Glycidylmethacrylat [%]** | | 0,42 % | n.b. | n.b. | n.b. | n.b. |

| | | | | | | |
|---|---|---|---|---|---|---|
| Fazit: Das zum Ende der Zugabe der Edukte mit einer molaren Stöchiometrie von 1:0,985 hergestellte Produkt erwies sich als NICHT lagerstabil! | | | | | | |

### Herstellungsbeispiel 11 (nicht erfindungsgemäß)

### Molare Stöchiometrie (Glycidylmethacrylat : Carbonsäure) 1:1

Verfahren dadurch gekennzeichnet, dass die Durchführung analog Beispiel 1 durchgeführt wird, jedoch als Carbonsäure 600,4 g Stearinsäure verwendet wird.

| **Lagerstabilität bei 30°C** | | **Start** | **1 Monat** | **3 Monate** | **6 Monate** | **8 Monate** |
|---|---|---|---|---|---|---|
| **Diestergehalt [%]** | min. 85,00 | 86,2 % | 81,7 % | 53,0 % | 48,8 % | 48,5 % |
| **Triestergehalt [%]** | max. 3,00 | 1,7 % | 5,7 % | 29,4 % | 34,9 % | 35,2 % |
| **Stearinsäure [%]** | | 0,74 % | n.b. | n.b. | n.b. | n.b. |
| **Glycidylmethacrylat [%]** | | 0,39 % | n.b. | n.b. | n.b. | n.b. |

| **Lagerstabilität bei 50°C** | | **Start** | **1 Monat** | **3 Monate** | **6 Monate** | **8 Monate** |
|---|---|---|---|---|---|---|
| **Diestergehalt [%]** | min. 85,00 | 86,2 % | 49,1 % | 46,9 % | 47,2 % | 47,1 % |
| **Triestergehalt [%]** | max. 3,00 | 1,7 % | 33,4 % | 35,1 % | 35,3 % | 35,1 % |
| **Stearinsäure [%]** | | 0,74 % | n.b. | n.b. | n.b. | n.b. |
| **Glycidylmethacrylat [%]** | | 0,39 % | n.b. | n.b. | n.b. | n.b. |

| | | | | | | |
|---|---|---|---|---|---|---|
| Fazit: Das zum Ende der Zugabe der Edukte mit einer molaren Stöchiometrie von 1:1 hergestellte Produkt erwies sich als NICHT lagerstabil! | | | | | | |

### Erfindungsgemäßes Beispiel 12

### Molare Stöchiometrie (Glycidylmethacrylat : Carbonsäure) 1:1,02

Verfahren dadurch gekennzeichnet, dass die Durchführung analog Beispiel 1 durchgeführt wird, jedoch als Carbonsäure 612,4 g Stearinsäure verwendet wird.

| **Lagerstabilität bei 30°C** | | **Start** | **1 Monat** | **3 Monate** | **6 Monate** | **8 Monate** |
|---|---|---|---|---|---|---|
| **Diestergehalt [%]** | min. 85,00 | 86,9 % | 87,6 % | 87,4 % | 87,5 % | 86,9 % |
| **Triestergehalt [%]** | max. 3,00 | 1,6 % | 1,7 % | 1,6 % | 1,8 % | 2,0 % |
| **Stearinsäure [%]** | | 0,79 % | n.b. | n.b. | n.b. | n.b. |
| **Glycidylmethacrylat [%]** | | 0,35 % | n.b. | n.b. | n.b. | n.b. |

| **Lagerstabilität bei 50°C** | | **Start** | **1 Monat** | **3 Monate** | **6 Monate** | **8 Monate** |
|---|---|---|---|---|---|---|
| **Diestergehalt [%]** | min. 85,00 | 86,9 % | 87,1 % | 86,9 % | 86,4 % | 84,9 % |
| **Triestergehalt [%]** | max. 3,00 | 1,6 % | 1,9 % | 2,2 % | 2,8 % | 3,3 % |
| **Stearinsäure [%]** | | 0,79 % | n.b. | n.b. | n.b. | n.b. |
| **Glycidylmethacrylat [%]** | | 0,35 % | n.b. | n.b. | n.b. | n.b. |

Fazit: Das zum Ende der Zugabe der Edukte mit einer molaren Stöchiometrie von 1:1,02 hergestellte Produkt hatte eine Lagerstabilität von über 6 Monaten.

### Herstellungsbeispiel 13 (nicht erfindungsgemäß)

Verfahren dadurch gekennzeichnet, dass die Durchführung analog Beispiel 1 durchgeführt wird, jedoch als Carbonsäure 253,9 g Benzoesäure verwendet wird.

### Molare Stöchiometrie (Glycidylmethacrylat : Carbonsäure) 1:0,985

| **Lagerstabilität bei 30°C** | | **Start** | **1 Monat** | **3 Monate** | **6 Monate** | **8 Monate** |
|---|---|---|---|---|---|---|
| **Diestergehalt [%]** | min. 85,00 | 86,2 % | 81,8 % | 53,1 % | 48,8 % | 48,5 % |
| **Triestergehalt [%]** | max. 3,00 | 1,7 % | 5,8 % | 30,1 % | 35,7 % | 36,1 % |
| **Benzoesäure [%]** | | 0,49 % | n.b. | n.b. | n.b. | n.b. |
| **Glycidylmethacrylat [%]** | | 0,68 % | n.b. | n.b. | n.b. | n.b. |

| **Lagerstabilität bei 50°C** | | **Start** | **1 Monat** | **3 Monate** | **6 Monate** | **8 Monate** |
|---|---|---|---|---|---|---|
| **Diestergehalt [%]** | min. 85,00 | 86,2 % | 49,1 % | 46,9 % | 47,2 % | 47,1 % |
| **Triestergehalt [%]** | max. 3,00 | 1,7 % | 34,2 % | 36,0 % | 36,2 % | 36,0 % |
| **Benzoesäure [%]** | | 0,49 % | n.b. | n.b. | n.b. | n.b. |
| **Glycidylmethacrylat [%]** | | 0,68 % | n.b. | n.b. | n.b. | n.b. |

| | | | | | | |
|---|---|---|---|---|---|---|
| Fazit: Das zum Ende der Zugabe der Edukte mit einer molaren Stöchiometrie von 1:0,985 hergestellte Produkt erwies sich als NICHT lagerstabil! | | | | | | |

### Herstellungsbeispiel 14 (nicht erfindungsgemäß)

### Molare Stöchiometrie (Glycidylmethacrylat : Carbonsäure) 1:1

Verfahren dadurch gekennzeichnet, dass die Durchführung analog Beispiel 1 durchgeführt wird, jedoch als Carbonsäure 257,7 g Benzoesäure verwendet wird.

| **Lagerstabilität bei 30°C** | | **Start** | **1 Monat** | **3 Monate** | **6 Monate** | **8 Monate** |
|---|---|---|---|---|---|---|
| **Diestergehalt [%]** | min. 85,00 | 88,1 % | 83,6 % | 54,3 % | 49,9 % | 49,6 % |
| **Triestergehalt [%]** | max. 3,00 | 1,6 % | 5,4 % | 27,8 % | 33,1 % | 33,4 % |
| **Benzoesäure [%]** | | 0,51 % | n.b. | n.b. | n.b. | n.b. |
| **Glycidylmethacrylat [%]** | | 0,62 % | n.b. | n.b. | n.b. | n.b. |

| **Lagerstabilität bei 50°C** | | **Start** | **1 Monat** | **3 Monate** | **6 Monate** | **8 Monate** |
|---|---|---|---|---|---|---|
| **Diestergehalt [%]** | min. 85,00 | 88,1 % | 50,2 % | 48,0 % | 48,3 % | 48,1 % |
| **Triestergehalt [%]** | max. 3,00 | 1,6 % | 31,7 % | 33,3 % | 33,5 % | 33,3 % |
| **Benzoesäure [%]** | | 0,51 % | n.b. | n.b. | n.b. | n.b. |
| **Glycidylmethacrylat [%]** | | 0,62 % | n.b. | n.b. | n.b. | n.b. |

| | | | | | | |
|---|---|---|---|---|---|---|
| Fazit: Das zum Ende der Zugabe der Edukte mit einer molaren Stöchiometrie von 1:1 hergestellte Produkt erwies sich als NICHT lagerstabil! | | | | | | |

### Erfindungsgemäßes Beispiel 15

### Molare Stöchiometrie (Glycidylmethacrylat : Carbonsäure) 1:1,02

Verfahren dadurch gekennzeichnet, dass die Durchführung analog Beispiel 1 durchgeführt wird, jedoch als Carbonsäure 262,9 g Benzoesäure verwendet wird.

| **Lagerstabilität bei 30°C** | | **Start** | **1 Monat** | **3 Monate** | **6 Monate** | **8 Monate** |
|---|---|---|---|---|---|---|
| **Diestergehalt [%]** | min. 85,00 | 88,3 % | 89,0 % | 88,8 % | 88,9 % | 88,3 % |
| **Triestergehalt [%]** | max. 3,00 | 1,2 % | 1,3 % | 1,2 % | 1,4 % | 1,6 % |
| **Benzoesäure [%]** | | 0,55 % | n.b. | n.b. | n.b. | n.b. |
| **Glycidylmethacrylat [%]** | | 0,56 % | n.b. | n.b. | n.b. | n.b. |

| **Lagerstabilität bei 50°C** | | **Start** | **1 Monat** | **3 Monate** | **6 Monate** | **8 Monate** |
|---|---|---|---|---|---|---|
| **Diestergehalt [%]** | min. 85,00 | 88,3 % | 88,5 % | 88,3 % | 87,8 % | 86,3 % |
| **Triestergehalt [%]** | max. 3,00 | 1,2 % | 1,5 % | 1,7 % | 2,2 % | 2,5 % |
| **Benzoesäure [%]** | | 0,55 % | n.b. | n.b. | n.b. | n.b. |
| **Glycidylmethacrylat [%]** | | 0,56 % | n.b. | n.b. | n.b. | n.b. |

| | | | | | | |
|---|---|---|---|---|---|---|
| Fazit: Das zum Ende der Zugabe der Edukte mit einer molaren Stöchiometrie von 1:1,02 hergestellte Produkt hatte eine Lagerstabilität von über 8 Monaten. | | | | | | |

### Herstellungsbeispiel 16 (nicht erfindungsgemäß)

Verfahren dadurch gekennzeichnet, dass die Durchführung analog Beispiel 1 durchgeführt wird, jedoch als Carbonsäure 287,1 g Salicylsäure und ein Lösungsmittel verwendet werden.

### Molare Stöchiometrie (Glycidylmethacrylat : Carbonsäure) 1:0,985

| **Lagerstabilität bei 30°C** | | **Start** | **1 Monat** | **3 Monate** | **6 Monate** | **8 Monate** |
|---|---|---|---|---|---|---|
| **Diestergehalt [%]** | min. 85,00 | 88,7 % | 84,2 % | 54,6 % | 50,2 % | 49,9 % |
| **Triestergehalt [%]** | max. 3,00 | 1,4 % | 4,7 % | 24,1 % | 28,6 % | 28,9 % |
| **Salicylsäure [%]** | | 0,52 % | n.b. | n.b. | n.b. | n.b. |
| **Glycidylmethacrylat [%]** | | 0,64 % | n.b. | n.b. | n.b. | n.b. |

| **Lagerstabilität bei 50°C** | | **Start** | **1 Monat** | **3 Monate** | **6 Monate** | **8 Monate** |
|---|---|---|---|---|---|---|
| **Diestergehalt [%]** | min. 85,00 | 88,7 % | 50,6 % | 48,3 % | 48,6 % | 48,5 % |
| **Triestergehalt [%]** | max. 3,00 | 1,4 % | 27,4 % | 28,8 % | 29,0 % | 28,8 % |
| **Salicylsäure [%]** | | 0,52 % | n.b. | n.b. | n.b. | n.b. |
| **Glycidylmethacrylat [%]** | | 0,64 % | n.b. | n.b. | n.b. | n.b. |

| | | | | | | |
|---|---|---|---|---|---|---|
| Fazit: Das zum Ende der Zugabe der Edukte mit einer molaren Stöchiometrie von 1:0,985 hergestellte Produkt erwies sich als NICHT lagerstabil! | | | | | | |

### Herstellungsbeispiel 17 (nicht erfindungsgemäß)

### Molare Stöchiometrie (Glycidylmethacrylat : Carbonsäure) 1:1

Verfahren dadurch gekennzeichnet, dass die Durchführung analog Beispiel 1 durchgeführt wird, jedoch als Carbonsäure 291,5 g Salicylsäure und ein Lösungsmittel verwendet werden.

| **Lagerstabilität bei 30°C** | | **Start** | **1 Monat** | **3 Monate** | **6 Monate** | **8 Monate** |
|---|---|---|---|---|---|---|
| **Diestergehalt [%]** | min. 85,00 | 89,6 % | 85,0 % | 55,2 % | 50,7 % | 50,4 % |
| **Triestergehalt [%]** | max. 3,00 | 1,8 % | 6,2 % | 32,0 % | 37,9 % | 38,4 % |
| **Salicylsäure [%]** | | 0,55 % | n.b. | n.b. | n.b. | n.b. |
| **Glycidylmethacrylat [%]** | | 0,59 % | n.b. | n.b. | n.b. | n.b. |

| **Lagerstabilität bei 50°C** | | **Start** | **1 Monat** | **3 Monate** | **6 Monate** | **8 Monate** |
|---|---|---|---|---|---|---|
| **Diestergehalt [%]** | min. 85,00 | 89,6 % | 51,1 % | 48,8 % | 49,1 % | 48,9 % |
| **Triestergehalt [%]** | max. 3,00 | 1,8 % | 36,4 % | 38,2 % | 38,5 % | 38,2 % |
| **Salicylsäure [%]** | | 0,55 % | n.b. | n.b. | n.b. | n.b. |
| **Glycidylmethacrylat [%]** | | 0,59 % | n.b. | n.b. | n.b. | n.b. |

| | | | | | | |
|---|---|---|---|---|---|---|
| Fazit: Das zum Ende der Zugabe der Edukte mit einer molaren Stöchiometrie von 1:1 hergestellte Produkt erwies sich als NICHT lagerstabil! | | | | | | |

### Erfindungsgemäßes Beispiel 18

### Molare Stöchiometrie (Glycidylmethacrylat : Carbonsäure) 1:1,02

Verfahren dadurch gekennzeichnet, dass die Durchführung analog Beispiel 1 durchgeführt wird, jedoch als Carbonsäure 297,3 g Salicylsäure und ein Lösungsmittel verwendet werden.

| **Lagerstabilität bei 30°C** | | **Start** | **1 Monat** | **3 Monate** | **6 Monate** | **8 Monate** |
|---|---|---|---|---|---|---|
| **Diestergehalt [%]** | min. 85,00 | 89,7 % | 90,4 % | 90,2 % | 90,4 % | 89,7 % |
| **Triestergehalt [%]** | max. 3,00 | 1,4 % | 1,5 % | 1,4 % | 1,6 % | 1,8 % |
| **Salicylsäure [%]** | | 0,59 % | n.b. | n.b. | n.b. | n.b. |
| **Glycidylmethacrylat [%]** | | 0,53 % | n.b. | n.b. | n.b. | n.b. |

| **Lagerstabilität bei 50°C** | | **Start** | **1 Monat** | **3 Monate** | **6 Monate** | **8 Monate** |
|---|---|---|---|---|---|---|
| **Diestergehalt [%]** | min. 85,00 | 89,7 % | 89,9 % | 89,7 % | 89,2 % | 87,7 % |
| **Triestergehalt [%]** | max. 3,00 | 1,4 % | 1,7 % | 2,0 % | 2,5 % | 2,9 % |
| **Salicylsäure [%]** | | 0,59 % | n.b. | n.b. | n.b. | n.b. |
| **Glycidylmethacrylat [%]** | | 0,53 % | n.b. | n.b. | n.b. | n.b. |

| | | | | | | |
|---|---|---|---|---|---|---|
| Fazit: Das zum Ende der Zugabe der Edukte mit einer molaren Stöchiometrie von 1:1,02 hergestellte Produkt hatte eine Lagerstabilität von über 8 Monaten. | | | | | | |

### Herstellungsbeispiel 19 (nicht erfindungsgemäß)

Verfahren dadurch gekennzeichnet, dass die Durchführung analog Beispiel 1 durchgeführt wird, jedoch als Carbonsäure 255,9 g Nicotinsäure und ein Lösungsmittel verwendet werden.

### Molare Stöchiometrie (Glycidylmethacrylat : Carbonsäure) 1:0,985

| **Lagerstabilität bei 30°C** | | **Start** | **1 Monat** | **3 Monate** | **6 Monate** | **8 Monate** |
|---|---|---|---|---|---|---|
| **Diestergehalt [%]** | min. 85,00 | 87,6 % | 83,1 % | 53,9 % | 49,6 % | 49,3 % |
| **Triestergehalt [%]** | max. 3,00 | 1,6 % | 5,4 % | 27,9 % | 33,1 % | 33,5 % |
| **Nicotinsäure [%]** | | 0,49 % | n.b. | n.b. | n.b. | n.b. |
| **Glycidylmethacrylat [%]** | | 0,67 % | n.b. | n.b. | n.b. | n.b. |

| **Lagerstabilität bei 50°C** | | **Start** | **1 Monat** | **3 Monate** | **6 Monate** | **8 Monate** |
|---|---|---|---|---|---|---|
| **Diestergehalt [%]** | min. 85,00 | 87,6 % | 49,9 % | 47,7 % | 48,0 % | 47,8 % |
| **Triestergehalt [%]** | max. 3,00 | 1,6 % | 31,7 % | 33,4 % | 33,5 % | 33,3 % |
| **Nicotinsäure [%]** | | 0,49 % | n.b. | n.b. | n.b. | n.b. |
| **Glycidylmethacrylat [%]** | | 0,67 % | n.b. | n.b. | n.b. | n.b. |

| | | | | | | |
|---|---|---|---|---|---|---|
| Fazit: Das zum Ende der Zugabe der Edukte mit einer molaren Stöchiometrie von 1:0,985 hergestellte Produkt erwies sich als NICHT lagerstabil! | | | | | | |

### Herstellungsbeispiel 20 (nicht erfindungsgemäß)

### Molare Stöchiometrie (Glycidylmethacrylat : Carbonsäure) 1:1

Verfahren dadurch gekennzeichnet, dass die Durchführung analog Beispiel 1 durchgeführt wird, jedoch als Carbonsäure 259,8 g Nicotinsäure und ein Lösungsmittel verwendet werden.

| **Lagerstabilität bei 30°C** | | **Start** | **1 Monat** | **3 Monate** | **6 Monate** | **8 Monate** |
|---|---|---|---|---|---|---|
| **Diestergehalt [%]** | min. 85,00 | 87,0 % | 82,5 % | 53,6 % | 49,2 % | 48,9 % |
| **Triestergehalt [%]** | max. 3,00 | 1,8 % | 5,9 % | 30,7 % | 36,5 % | 36,9 % |
| **Nicotinsäure [%]** | | 0,51 % | n.b. | n.b. | n.b. | n.b. |
| **Glycidylmethacrylat [%]** | | 0,62 % | n.b. | n.b. | n.b. | n.b. |

| **Lagerstabilität bei 50°C** | | **Start** | **1 Monat** | **3 Monate** | **6 Monate** | **8 Monate** |
|---|---|---|---|---|---|---|
| **Diestergehalt [%]** | min. 85,00 | 87,0 % | 49,6 % | 47,3 % | 47,7 % | 47,5 % |
| **Triestergehalt [%]** | max. 3,00 | 1,8 % | 35,0 % | 36,8 % | 37,0 % | 36,7 % |
| **Nicotinsäure [%]** | | 0,51 % | n.b. | n.b. | n.b. | n.b. |
| **Glycidylmethacrylat [%]** | | 0,62 % | n.b. | n.b. | n.b. | n.b. |

| | | | | | | |
|---|---|---|---|---|---|---|
| Fazit: Das zum Ende der Zugabe der Edukte mit einer molaren Stöchiometrie von 1:1 hergestellte Produkt erwies sich als NICHT lagerstabil! | | | | | | |

### Erfindungsgemäßes Beispiel 21

### Molare Stöchiometrie (Glycidylmethacrylat : Carbonsäure) 1:1,02

Verfahren dadurch gekennzeichnet, dass die Durchführung analog Beispiel 1 durchgeführt wird, jedoch als Carbonsäure 265 g Nicotinsäure und ein Lösungsmittel verwendet werden.

| **Lagerstabilität bei 30°C** | | **Start** | **1 Monat** | **3 Monate** | **6 Monate** | **8 Monate** |
|---|---|---|---|---|---|---|
| **Diestergehalt [%]** | min. 85,00 | 89,2 % | 89,9 % | 89,7 % | 89,8 % | 89,2 % |
| **Triestergehalt [%]** | max. 3,00 | 2,0 % | 2,0 % | 2,0 % | 2,2 % | 2,5 % |
| **Nicotinsäure [%]** | | 0,55 % | n.b. | n.b. | n.b. | n.b. |
| **Glycidylmethacrylat [%]** | | 0,56 % | n.b. | n.b. | n.b. | n.b. |

| **Lagerstabilität bei 50°C** | | **Start** | **1 Monat** | **3 Monate** | **6 Monate** | **8 Monate** |
|---|---|---|---|---|---|---|
| **Diestergehalt [%]** | min. 85,00 | 89,2 % | 89,3 % | 89,2 % | 88,6 % | 87,1 % |
| **Triestergehalt [%]** | max. 3,00 | 2,0 % | 2,3 % | 2,7 % | 3,0 % | 3,7 % |
| **Nicotinsäure [%]** | | 0,55 % | n.b. | n.b. | n.b. | n.b. |
| **Glycidylmethacrylat [%]** | | 0,56 % | n.b. | n.b. | n.b. | n.b. |

| | | | | | | |
|---|---|---|---|---|---|---|
| Fazit: Das zum Ende der Zugabe der Edukte mit einer molaren Stöchiometrie von 1:1,02 hergestellte Produkt hatte eine Lagerstabilität von über 6 Monaten. | | | | | | |

### Herstellungsbeispiel 22 (nicht erfindungsgemäß)

Verfahren dadurch gekennzeichnet, dass die Durchführung analog Beispiel 1 durchgeführt wird, jedoch als Carbonsäure 239,3 g Prolin und ein Lösungsmittel verwendet werden.

### Molare Stöchiometrie (Glycidylmethacrylat : Carbonsäure 1:0,985

| **Lagerstabilität bei 30°C** | | **Start** | **1 Monat** | **3 Monate** | **6 Monate** | **8 Monate** |
|---|---|---|---|---|---|---|
| **Diestergehalt [%]** | min. 85,00 | 88,6 % | 84,1 % | 54,6 % | 50,2 % | 49,9 % |
| **Triestergehalt [%]** | max. 3,00 | 1,9 % | 6,3 % | 32,6 % | 38,7 % | 39,1 % |
| **Prolin [%]** | | 0,47 % | n.b. | n.b. | n.b. | n.b. |
| **Glycidylmethacrylat [%]** | | 0,70 % | n.b. | n.b. | n.b. | n.b. |

| **Lagerstabilität bei 50°C** | | **Start** | **1 Monat** | **3 Monate** | **6 Monate** | **8 Monate** |
|---|---|---|---|---|---|---|
| **Diestergehalt [%]** | min. 85,00 | 88,6 % | 50,5 % | 48,2 % | 48,6 % | 48,4 % |
| **Triestergehalt [%]** | max. 3,00 | 1,9 % | 37,1 % | 39,0 % | 39,2 % | 39,0 % |
| **Prolin [%]** | | 0,47 % | n.b. | n.b. | n.b. | n.b. |
| **Glycidylmethacrylat [%]** | | 0,70 % | n.b. | n.b. | n.b. | n.b. |

| | | | | | | |
|---|---|---|---|---|---|---|
| Fazit: Das zum Ende der Zugabe der Edukte mit einer molaren Stöchiometrie von 1:0,985 hergestellte Produkt erwies sich als NICHT lagerstabil! | | | | | | |

### Herstellungsbeispiel 23 (nicht erfindungsgemäß)

### Molare Stöchiometrie (Glycidylmethacrylat : Carbonsäure) 1:1

Verfahren dadurch gekennzeichnet, dass die Durchführung analog Beispiel 1 durchgeführt wird, jedoch als Carbonsäure 243 g Prolin und ein Lösungsmittel verwendet werden.

| **Lagerstabilität bei 30°C** | | **Start** | **1 Monat** | **3 Monate** | **6 Monate** | **8 Monate** |
|---|---|---|---|---|---|---|
| **Diestergehalt [%]** | min. 85,00 | 89,0 % | 84,4 % | 54,8 % | 50,4 % | 50,1 % |
| **Triestergehalt [%]** | max. 3,00 | 1,8 % | 5,9 % | 30,3 % | 36,0 % | 36,3 % |
| **Prolin [%]** | | 0,50 % | n.b. | n.b. | n.b. | n.b. |
| **Glycidylmethacrylat [%]** | | 0,64 % | n.b. | n.b. | n.b. | n.b. |

| **Lagerstabilität bei 50°C** | | **Start** | **1 Monat** | **3 Monate** | **6 Monate** | **8 Monate** |
|---|---|---|---|---|---|---|
| **Diestergehalt [%]** | min. 85,00 | 89,0 % | 50,7 % | 48,4 % | 48,8 % | 48,6 % |
| **Triestergehalt [%]** | max. 3,00 | 1,8 % | 34,5 % | 36,2 % | 36,4 % | 36,2 % |
| **Prolin [%]** | | 0,50 % | n.b. | n.b. | n.b. | n.b. |
| **Glycidylmethacrylat [%]** | | 0,64 % | n.b. | n.b. | n.b. | n.b. |

| | | | | | | |
|---|---|---|---|---|---|---|
| Fazit: Das zum Ende der Zugabe der Edukte mit einer molaren Stöchiometrie von 1:1 hergestellte Produkt erwies sich als NICHT lagerstabil! | | | | | | |

### Erfindungsgemäßes Beispiel 24

### Molare Stöchiometrie (Glycidylmethacrylat : Carbonsäure) 1:1,02

Verfahren dadurch gekennzeichnet, dass die Durchführung analog Beispiel 1 durchgeführt wird, jedoch als Carbonsäure 247,8 g Prolin und ein Lösungsmittel verwendet werden.

| **Lagerstabilität bei 30°C** | | **Start** | **1 Monat** | **3 Monate** | **6 Monate** | **8 Monate** |
|---|---|---|---|---|---|---|
| **Diestergehalt [%]** | min. 85,00 | 85,6 % | 86,3 % | 86,1 % | 86,2 % | 85,6 % |
| **Triestergehalt [%]** | max. 3,00 | 1,1 % | 1,1 % | 1,1 % | 1,2 % | 1,4 % |
| **Prolin [%]** | | 0,53 % | n.b. | n.b. | n.b. | n.b. |
| **Glycidylmethacrylat [%]** | | 0,58 % | n.b. | n.b. | n.b. | n.b. |

| **Lagerstabilität bei 50°C** | | **Start** | **1 Monat** | **3 Monate** | **6 Monate** | **8 Monate** |
|---|---|---|---|---|---|---|
| **Diestergehalt [%]** | min. 85,00 | 85,6 % | 85,7 % | 85,6 % | 85,1 % | 83,6 % |
| **Triestergehalt [%]** | max. 3,00 | 1,1 % | 1,3 % | 1,5 % | 1,9 % | 2,2 % |
| **Prolin [%]** | | 0,53 % | n.b. | n.b. | n.b. | n.b. |
| **Glycidylmethacrylat [%]** | | 0,58 % | n.b. | n.b. | n.b. | n.b. |

| | | | | | | |
|---|---|---|---|---|---|---|
| Fazit: Das zum Ende der Zugabe der Edukte mit einer molaren Stöchiometrie von 1:1,02 hergestellte Produkt hatte eine Lagerstabilität von über 6 Monaten. | | | | | | |

### Herstellungsbeispiel 25 (nicht erfindungsgemäß)

Verfahren dadurch gekennzeichnet, dass die Durchführung analog Beispiel 1 durchgeführt wird, jedoch als Carbonsäure 149,8 g Acrylsäure verwendet wird.

### Molare Stöchiometrie (Glycidylmethacrylat : Carbonsäure) 1:0,985

| **Lagerstabilität bei 30°C** | | **Start** | **1 Monat** | **3 Monate** | **6 Monate** | **8 Monate** |
|---|---|---|---|---|---|---|
| **Diestergehalt [%]** | min. 85,00 | 89,0 % | 84,4 % | 54,8 % | 50,4 % | 50,1 % |
| **Triestergehalt [%]** | max. 3,00 | 1,4 % | 4,6 % | 23,8 % | 28,3 % | 28,6 % |
| **Acrylsäure [%]** | | 0,36 % | n.b. | n.b. | n.b. | n.b. |
| **Glycidylmethacrylat [%]** | | 0,83 % | n.b. | n.b. | n.b. | n.b. |

| **Lagerstabilität bei 50°C** | | **Start** | **1 Monat** | **3 Monate** | **6 Monate** | **8 Monate** |
|---|---|---|---|---|---|---|
| **Diestergehalt [%]** | min. 85,00 | 89,0 % | 50,7 % | 48,4 % | 48,8 % | 48,6 % |
| **Triestergehalt [%]** | max. 3,00 | 1,4 % | 27,1 % | 28,5 % | 28,7 % | 28,5 % |
| **Acrylsäure [%]** | | 0,36 % | n.b. | n.b. | n.b. | n.b. |
| **Glycidylmethacrylat [%]** | | 0,83 % | n.b. | n.b. | n.b. | n.b. |

| | | | | | | |
|---|---|---|---|---|---|---|
| Fazit: Das zum Ende der Zugabe der Edukte mit einer molaren Stöchiometrie von 1:0,985 hergestellte Produkt erwies sich als NICHT lagerstabil! | | | | | | |

### Herstellungsbeispiel 26 (nicht erfindungsgemäß)

### Molare Stöchiometrie (Glycidylmethacrylat : Carbonsäure) 1:1

Verfahren dadurch gekennzeichnet, dass die Durchführung analog Beispiel 1 durchgeführt wird, jedoch als Carbonsäure 152,1 g Acrylsäure verwendet wird.

| **Lagerstabilität bei 30°C** | | **Start** | **1 Monat** | **3 Monate** | **6 Monate** | **8 Monate** |
|---|---|---|---|---|---|---|
| **Diestergehalt [%]** | min. 85,00 | 88,2 % | 83,7 % | 54,3 % | 49,9 % | 49,6 % |
| **Triestergehalt [%]** | max. 3,00 | 1,8 % | 6,0 % | 30,9 % | 36,7 % | 37,1 % |
| **Acrylsäure [%]** | | 0,37 % | n.b. | n.b. | n.b. | n.b. |
| **Glycidylmethacrylat [%]** | | 0,77 % | n.b. | n.b. | n.b. | n.b. |

| **Lagerstabilität bei 50°C** | | **Start** | **1 Monat** | **3 Monate** | **6 Monate** | **8 Monate** |
|---|---|---|---|---|---|---|
| **Diestergehalt [%]** | min. 85,00 | 88,2 % | 50,3 % | 48,0 % | 48,3 % | 48,2 % |
| **Triestergehalt [%]** | max. 3,00 | 1,8 % | 35,2 % | 37,0 % | 37,2 % | 36,9 % |
| **Acrylsäure [%]** | | 0,37 % | n.b. | n.b. | n.b. | n.b. |
| **Glycidylmethacrylat [%]** | | 0,77 % | n.b. | n.b. | n.b. | n.b. |

| | | | | | | |
|---|---|---|---|---|---|---|
| Fazit: Das zum Ende der Zugabe der Edukte mit einer molaren Stöchiometrie von 1:1 hergestellte Produkt erwies sich als NICHT lagerstabil! | | | | | | |

### Erfindungsgemäßes Beispiel 27

### Molare Stöchiometrie (Glycidylmethacrylat : Carbonsäure) 1:1,02

Verfahren dadurch gekennzeichnet, dass die Durchführung analog Beispiel 1 durchgeführt wird, jedoch als Carbonsäure 155,1 g Acrylsäure verwendet wird.

| **Lagerstabilität bei 30°C** | | **Start** | **1 Monat** | **3 Monate** | **6 Monate** | **8 Monate** |
|---|---|---|---|---|---|---|
| **Diestergehalt [%]** | min. 85,00 | 88,3 % | 89,0 % | 88,8 % | 88,9 % | 88,3 % |
| **Triestergehalt [%]** | max. 3,00 | 1,8 % | 1,9 % | 1,8 % | 2,0 % | 2,3 % |
| **Acrylsäure [%]** | | 0,40 % | n.b. | n.b. | n.b. | n.b. |
| **Glycidylmethacrylat [%]** | | 0,69 % | n.b. | n.b. | n.b. | n.b. |

| **Lagerstabilität bei 50°C** | | **Start** | **1 Monat** | **3 Monate** | **6 Monate** | **8 Monate** |
|---|---|---|---|---|---|---|
| **Diestergehalt [%]** | min. 85,00 | 88,3 % | 88,4 % | 88,3 % | 87,7 % | 86,2 % |
| **Triestergehalt [%]** | max. 3,00 | 1,8 % | 2,2 % | 2,4 % | 2,7 % | 3,0 % |
| **Acrylsäure [%]** | | 0,40 % | n.b. | n.b. | n.b. | n.b. |
| **Glycidylmethacrylat [%]** | | 0,69 % | n.b. | n.b. | n.b. | n.b. |

| | | | | | | |
|---|---|---|---|---|---|---|
| Fazit: Das zum Ende der Zugabe der Edukte mit einer molaren Stöchiometrie von 1:1,02 hergestellte Produkt hatte eine Lagerstabilität von über 8 Monaten. | | | | | | |

### Herstellungsbeispiel 28 (nicht erfindungsgemäß)

Verfahren dadurch gekennzeichnet, dass die Durchführung analog Beispiel 1 durchgeführt wird, jedoch als Carbonsäure 587,2 g Ölsäure verwendet wird.

### Molare Stöchiometrie (Glycidylmethacrylat : Carbonsäure) 1:0,985

| **Lagerstabilität bei 30°C** | | **Start** | **1 Monat** | **3 Monate** | **6 Monate** | **8 Monate** |
|---|---|---|---|---|---|---|
| **Diestergehalt [%]** | min. 85,00 | 87,6 % | 83,1 % | 53,9 % | 49,6 % | 49,3 % |
| **Triestergehalt [%]** | max. 3,00 | 1,2 % | 4,1 % | 21,4 % | 25,4 % | 25,6 % |
| **Ölsäure [%]** | | 0,71 % | n.b. | n.b. | n.b. | n.b. |
| **Glycidylmethacrylat [%]** | | 0,42 % | n.b. | n.b. | n.b. | n.b. |

| **Lagerstabilität bei 50°C** | | **Start** | **1 Monat** | **3 Monate** | **6 Monate** | **8 Monate** |
|---|---|---|---|---|---|---|
| **Diestergehalt [%]** | min. 85,00 | 87,6 % | 49,9 % | 47,7 % | 48,0 % | 47,8 % |
| **Triestergehalt [%]** | max. 3,00 | 1,2 % | 24,3 % | 25,6 % | 25,7 % | 25,5 % |
| **Ölsäure [%]** | | 0,71 % | n.b. | n.b. | n.b. | n.b. |
| **Glycidylmethacrylat [%]** | | 0,42 % | n.b. | n.b. | n.b. | n.b. |

| | | | | | | |
|---|---|---|---|---|---|---|
| Fazit: Das zum Ende der Zugabe der Edukte mit einer molaren Stöchiometrie von 1:0,985 hergestellte Produkt erwies sich als NICHT lagerstabil! | | | | | | |

### Herstellungsbeispiel 29 (nicht erfindungsgemäß)

### Molare Stöchiometrie (Glycidylmethacrylat : Carbonsäure) 1:1

Verfahren dadurch gekennzeichnet, dass die Durchführung analog Beispiel 1 durchgeführt wird, jedoch als Carbonsäure 596,1 g Ölsäure verwendet wird.

| **Lagerstabilität bei 30°C** | | **Start** | **1 Monat** | **3 Monate** | **6 Monate** | **8 Monate** |
|---|---|---|---|---|---|---|
| **Diestergehalt [%]** | min. 85,00 | 89,1 % | 84,6 % | 54,9 % | 50,4 % | 50,2 % |
| **Triestergehalt [%]** | max. 3,00 | 1,1 % | 3,8 % | 19,7 % | 23,4 % | 23,7 % |
| **Ölsäure [%]** | | 0,74 % | n.b. | n.b. | n.b. | n.b. |
| **Glycidylmethacrylat [%]** | | 0,39 % | n.b. | n.b. | n.b. | n.b. |

| **Lagerstabilität bei 50°C** | | **Start** | **1 Monat** | **3 Monate** | **6 Monate** | **8 Monate** |
|---|---|---|---|---|---|---|
| **Diestergehalt [%]** | min. 85,00 | 89,1 % | 50,8 % | 48,5 % | 48,8 % | 48,7 % |
| **Triestergehalt [%]** | max. 3,00 | 1,1 % | 22,5 % | 23,6 % | 23,8 % | 23,6 % |
| **Ölsäure [%]** | | 0,74 % | n.b. | n.b. | n.b. | n.b. |
| **Glycidylmethacrylat [%]** | | 0,39 % | n.b. | n.b. | n.b. | n.b. |

| | | | | | | |
|---|---|---|---|---|---|---|
| Fazit: Das zum Ende der Zugabe der Edukte mit einer molaren Stöchiometrie von 1:1 hergestellte Produkt erwies sich als NICHT lagerstabil! | | | | | | |

### Erfindungsgemäßes Beispiel 30

### Molare Stöchiometrie (Glycidylmethacrylat : Carbonsäure) 1:1,02

Verfahren dadurch gekennzeichnet, dass die Durchführung analog Beispiel 1 durchgeführt wird, jedoch als Carbonsäure 608 g Ölsäure verwendet wird.

| **Lagerstabilität bei 30°C** | | **Start** | **1 Monat** | **3 Monate** | **6 Monate** | **8 Monate** |
|---|---|---|---|---|---|---|
| **Diestergehalt [%]** | min. 85,00 | 87,7 % | 88,4 % | 88,2 % | 88,3 % | 87,7 % |
| **Triestergehalt [%]** | max. 3,00 | 1,4 % | 1,4 % | 1,4 % | 1,5 % | 1,8 % |
| **Ölsäure [%]** | | 0,79 % | n.b. | n.b. | n.b. | n.b. |
| **Glycidylmethacrylat [%]** | | 0,35 % | n.b. | n.b. | n.b. | n.b. |

| **Lagerstabilität bei 50°C** | | **Start** | **1 Monat** | **3 Monate** | **6 Monate** | **8 Monate** |
|---|---|---|---|---|---|---|
| **Diestergehalt [%]** | min. 85,00 | 87,7 % | 87,8 % | 87,7 % | 87,1 % | 85,7 % |
| **Triestergehalt [%]** | max. 3,00 | 1,4 % | 1,7 % | 1,9 % | 2,4 % | 2,8 % |
| **Ölsäure [%]** | | 0,79 % | n.b. | n.b. | n.b. | n.b. |
| **Glycidylmethacrylat [%]** | | 0,35 % | n.b. | n.b. | n.b. | n.b. |

| | | | | | | |
|---|---|---|---|---|---|---|
| Fazit: Das zum Ende der Zugabe der Edukte mit einer molaren Stöchiometrie von 1:1,02 hergestellte Produkt hatte eine Lagerstabilität von über 8 Monaten. | | | | | | |

### Herstellungsbeispiel 31 (nicht erfindungsgemäß)

Verfahren dadurch gekennzeichnet, dass die Durchführung analog Beispiel 1 durchgeführt wird, jedoch als Carbonsäure 187,3 g Milchsäure verwendet wird.

### Molare Stöchiometrie (Glycidylmethacrylat : Carbonsäure) 1:0,985

| **Lagerstabilität bei 30°C** | | **Start** | **1 Monat** | **3 Monate** | **6 Monate** | **8 Monate** |
|---|---|---|---|---|---|---|
| **Diestergehalt [%]** | min. 85,00 | 89,0 % | 84,4 % | 54,8 % | 50,4 % | 50,1 % |
| **Triestergehalt [%]** | max. 3,00 | 1,0 % | 3,5 % | 18,0 % | 21,4 % | 21,7 % |
| **Milchsäure [%]** | | 0,41 % | n.b. | n.b. | n.b. | n.b. |
| **Glycidylmethacrylat [%]** | | 0,77 % | n.b. | n.b. | n.b. | n.b. |

| **Lagerstabilität bei 50°C** | | **Start** | **1 Monat** | **3 Monate** | **6 Monate** | **8 Monate** |
|---|---|---|---|---|---|---|
| **Diestergehalt [%]** | min. 85,00 | 89,0 % | 50,7 % | 48,4 % | 48,8 % | 48,6 % |
| **Triestergehalt [%]** | max. 3,00 | 1,0 % | 20,5 % | 21,6 % | 21,7 % | 21,6 % |
| **Milchsäure [%]** | | 0,41 % | n.b. | n.b. | n.b. | n.b. |
| **Glycidylmethacrylat [%]** | | 0,77 % | n.b. | n.b. | n.b. | n.b. |

| | | | | | | |
|---|---|---|---|---|---|---|
| Fazit: Das zum Ende der Zugabe der Edukte mit einer molaren Stöchiometrie von 1:0,985 hergestellte Produkt erwies sich als NICHT lagerstabil! | | | | | | |

### Herstellungsbeispiel 32 (nicht erfindungsgemäß)

### Molare Stöchiometrie (Glycidylmethacrylat : Carbonsäure) 1:1

Verfahren dadurch gekennzeichnet, dass die Durchführung analog Beispiel 1 durchgeführt wird, jedoch als Carbonsäure 190,1 g Milchsäure verwendet wird.

| **Lagerstabilität bei 30°C** | | **Start** | **1 Monat** | **3 Monate** | **6 Monate** | **8 Monate** |
|---|---|---|---|---|---|---|
| **Diestergehalt [%]** | min. 85,00 | 89,3 % | 84,8 % | 55,0 % | 50,6 % | 50,3 % |
| **Triestergehalt [%]** | max. 3,00 | 1,6 % | 5,2 % | 27,0 % | 32,1 % | 32,4 % |
| **Milchsäure [%]** | | 0,43 % | n.b. | n.b. | n.b. | n.b. |
| **Glycidylmethacrylat [%]** | | 0,71 % | n.b. | n.b. | n.b. | n.b. |

| **Lagerstabilität bei 50°C** | | **Start** | **1 Monat** | **3 Monate** | **6 Monate** | **8 Monate** |
|---|---|---|---|---|---|---|
| **Diestergehalt [%]** | min. 85,00 | 89,3 % | 50,9 % | 48,6 % | 49,0 % | 48,8 % |
| **Triestergehalt [%]** | max. 3,00 | 1,6 % | 30,8 % | 32,3 % | 32,5 % | 32,3 % |
| **Milchsäure [%]** | | 0,43 % | n.b. | n.b. | n.b. | n.b. |
| **Glycidylmethacrylat [%]** | | 0,71 % | n.b. | n.b. | n.b. | n.b. |

| | | | | | | |
|---|---|---|---|---|---|---|
| Fazit: Das zum Ende der Zugabe der Edukte mit einer molaren Stöchiometrie von 1:1 hergestellte Produkt erwies sich als NICHT lagerstabil! | | | | | | |

### Erfindungsgemäßes Beispiel 33

### Molare Stöchiometrie (Glycidylmethacrylat : Carbonsäure) 1:1,02

Verfahren dadurch gekennzeichnet, dass die Durchführung analog Beispiel 1 durchgeführt wird, jedoch als Carbonsäure 193,9 g Milchsäure verwendet wird.

| **Lagerstabilität bei 30°C** | | **Start** | **1 Monat** | **3 Monate** | **6 Monate** | **8 Monate** |
|---|---|---|---|---|---|---|
| **Diestergehalt [%]** | min. 85,00 | 85,6 % | 86,3 % | 86,1 % | 86,2 % | 85,6 % |
| **Triestergehalt [%]** | max. 3,00 | 1,4 % | 1,4 % | 1,4 % | 1,5 % | 1,7 % |
| **Milchsäure [%]** | | 0,46 % | n.b. | n.b. | n.b. | n.b. |
| **Glycidylmethacrylat [%]** | | 0,64 % | n.b. | n.b. | n.b. | n.b. |

| **Lagerstabilität bei 50°C** | | **Start** | **1 Monat** | **3 Monate** | **6 Monate** | **8 Monate** |
|---|---|---|---|---|---|---|
| **Diestergehalt [%]** | min. 85,00 | 85,6 % | 85,7 % | 85,6 % | 85,1 % | 83,6 % |
| **Triestergehalt [%]** | max. 3,00 | 1,4 % | 1,6 % | 1,9 % | 2,4 % | 2,8 % |
| **Milchsäure [%]** | | 0,46 % | n.b. | n.b. | n.b. | n.b. |
| **Glycidylmethacrylat [%]** | | 0,64 % | n.b. | n.b. | n.b. | n.b. |

| | | | | | | |
|---|---|---|---|---|---|---|
| Fazit: Das zum Ende der Zugabe der Edukte mit einer molaren Stöchiometrie von 1:1,02 hergestellte Produkt hatte eine Lagerstabilität von über 6 Monaten. | | | | | | |

### Herstellungsbeispiel 34 (nicht erfindungsgemäß)

Verfahren dadurch gekennzeichnet, dass die Durchführung analog Beispiel 1 durchgeführt wird, jedoch als Carbonsäure 216,4 g Hydroxyisobuttersäure verwendet wird.

### Molare Stöchiometrie (Glycidylmethacrylat : Carbonsäure) 1:0,985

| **Lagerstabilität bei 30°C** | | **Start** | **1 Monat** | **3 Monate** | **6 Monate** | **8 Monate** |
|---|---|---|---|---|---|---|
| **Diestergehalt [%]** | min. 85,00 | 87,0 % | 82,5 % | 53,6 % | 49,2 % | 48,9 % |
| **Triestergehalt [%]** | max. 3,00 | 1,7 % | 5,8 % | 30,2 % | 35,9 % | 36,2 % |
| **Hydroxyisobuttersäure [%]** | | 0,45 % | n.b. | n.b. | n.b. | n.b. |
| **Glycidylmethacrylat [%]** | | 0,73 % | n.b. | n.b. | n.b. | n.b. |

| **Lagerstabilität bei 50°C** | | **Start** | **1 Monat** | **3 Monate** | **6 Monate** | **8 Monate** |
|---|---|---|---|---|---|---|
| **Diestergehalt [%]** | min. 85,00 | 87,0 % | 49,6 % | 47,4 % | 47,7 % | 47,5 % |
| **Triestergehalt [%]** | max. 3,00 | 1,7 % | 34,4 % | 36,1 % | 36,3 % | 36,1 % |
| **Hydroxyisobuttersäure [%]** | | 0,45 % | n.b. | n.b. | n.b. | n.b. |
| **Glycidylmethacrylat [%]** | | 0,73 % | n.b. | n.b. | n.b. | n.b. |

| | | | | | | |
|---|---|---|---|---|---|---|
| Fazit: Das zum Ende der Zugabe der Edukte mit einer molaren Stöchiometrie von 1:0,985 hergestellte Produkt erwies sich als NICHT lagerstabil! | | | | | | |

### Herstellungsbeispiel 35 (nicht erfindungsgemäß)

### Molare Stöchiometrie (Glycidylmethacrylat : Carbonsäure) 1:1

Verfahren dadurch gekennzeichnet, dass die Durchführung analog Beispiel 1 durchgeführt wird, jedoch als Carbonsäure 219,7 g Hydroxyisobuttersäure verwendet wird.

| **Lagerstabilität bei 30°C** | | **Start** | **1 Monat** | **3 Monate** | **6 Monate** | **8 Monate** |
|---|---|---|---|---|---|---|
| **Diestergehalt [%]** | min. 85,00 | 87,4 % | 83,0 % | 53,8 % | 49,5 % | 49,2 % |
| **Triestergehalt [%]** | max. 3,00 | 1,3 % | 4,4 % | 22,6 % | 26,8 % | 27,1 % |
| **Hydroxyisobuttersäure [%]** | | 0,47 % | n.b. | n.b. | n.b. | n.b. |
| **Glycidylmethacrylat [%]** | | 0,67 % | n.b. | n.b. | n.b. | n.b. |

| **Lagerstabilität bei 50°C** | | **Start** | **1 Monat** | **3 Monate** | **6 Monate** | **8 Monate** |
|---|---|---|---|---|---|---|
| **Diestergehalt [%]** | min. 85,00 | 87,4 % | 49,8 % | 47,6 % | 47,9 % | 47,8 % |
| **Triestergehalt [%]** | max. 3,00 | 1,3 % | 25,7 % | 27,0 % | 27,2 % | 27,0 % |
| **Hydroxyisobuttersäure [%]** | | 0,47 % | n.b. | n.b. | n.b. | n.b. |
| **Glycidylmethacrylat [%]** | | 0,67 % | n.b. | n.b. | n.b. | n.b. |

| | | | | | | |
|---|---|---|---|---|---|---|
| Fazit: Das zum Ende der Zugabe der Edukte mit einer molaren Stöchiometrie von 1:1 hergestellte Produkt erwies sich als NICHT lagerstabil! | | | | | | |

### Erfindungsgemäßes Beispiel 36

### Molare Stöchiometrie (Glycidylmethacrylat : Carbonsäure) 1:1,02

Verfahren dadurch gekennzeichnet, dass die Durchführung analog Beispiel 1 durchgeführt wird, jedoch als Carbonsäure 224,1 g Hydroxyisobuttersäure verwendet wird.

| **Lagerstabilität bei 30°C** | | **Start** | **1 Monat** | **3 Monate** | **6 Monate** | **8 Monate** |
|---|---|---|---|---|---|---|
| **Diestergehalt [%]** | min. 85,00 | 88,1 % | 88,8 % | 88,6 % | 88,7 % | 88,1 % |
| **Triestergehalt [%]** | max. 3,00 | 1,7 % | 1,7 % | 1,7 % | 1,9 % | 2,1 % |
| **Hydroxyisobuttersäure [%]** | | 0,50 % | n.b. | n.b. | n.b. | n.b. |
| **Glycidylmethacrylat [%]** | | 0,60 % | n.b. | n.b. | n.b. | n.b. |

| **Lagerstabilität bei 50°C** | | **Start** | **1 Monat** | **3 Monate** | **6 Monate** | **8 Monate** |
|---|---|---|---|---|---|---|
| **Diestergehalt [%]** | min. 85,00 | 88,1 % | 88,2 % | 88,1 % | 87,5 % | 86,0 % |
| **Triestergehalt [%]** | max. 3,00 | 1,7 % | 2,0 % | 2,3 % | 3,0 % | 3,4 % |
| **Hydroxyisobuttersäure [%]** | | 0,50 % | n.b. | n.b. | n.b. | n.b. |
| **Glycidylmethacrylat [%]** | | 0,60 % | n.b. | n.b. | n.b. | n.b. |

| | | | | | | |
|---|---|---|---|---|---|---|
| Fazit: Das zum Ende der Zugabe der Edukte mit einer molaren Stöchiometrie von 1:1,02 hergestellte Produkt hatte eine Lagerstabilität von über 6 Monaten. | | | | | | |

### Herstellungsbeispiel 37 (nicht erfindungsgemäß)

Verfahren dadurch gekennzeichnet, dass die Durchführung analog Beispiel 1 durchgeführt wird, jedoch als Carbonsäure 339,7 g Trichloressigsäure verwendet wird.

### Molare Stöchiometrie (Glycidylmethacrylat : Carbonsäure) 1:0,985

| **Lagerstabilität bei 30°C** | | **Start** | **1 Monat** | **3 Monate** | **6 Monate** | **8 Monate** |
|---|---|---|---|---|---|---|
| **Diestergehalt [%]** | min. 85,00 | 87,5 % | 83,0 % | 53,9 % | 49,5 % | 49,2 % |
| **Triestergehalt [%]** | max. 3,00 | 1,4 % | 4,8 % | 25,0 % | 29,7 % | 30,0 % |
| **Trichloressigsäure [%]** | | 0,57 % | n.b. | n.b. | n.b. | n.b. |
| **Glycidylmethacrylat [%]** | | 0,59 % | n.b. | n.b. | n.b. | n.b. |

| **Lagerstabilität bei 50°C** | | **Start** | **1 Monat** | **3 Monate** | **6 Monate** | **8 Monate** |
|---|---|---|---|---|---|---|
| **Diestergehalt [%]** | min. 85,00 | 87,5 % | 49,8 % | 47,6 % | 47,9 % | 47,8 % |
| **Triestergehalt [%]** | max. 3,00 | 1,4 % | 28,4 % | 29,9 % | 30,1 % | 29,9 % |
| **Trichloressigsäure [%]** | | 0,57 % | n.b. | n.b. | n.b. | n.b. |
| **Glycidylmethacrylat [%]** | | 0,59 % | n.b. | n.b. | n.b. | n.b. |

| | | | | | | |
|---|---|---|---|---|---|---|
| Fazit: Das zum Ende der Zugabe der Edukte mit einer molaren Stöchiometrie von 1:0,985 hergestellte Produkt erwies sich als NICHT lagerstabil! | | | | | | |

### Herstellungsbeispiel 38 (nicht erfindungsgemäß)

### Molare Stöchiometrie (Glycidylmethacrylat : Carbonsäure) 1:1

Verfahren dadurch gekennzeichnet, dass die Durchführung analog Beispiel 1 durchgeführt wird, jedoch als Carbonsäure 344,8 g Trichloressigsäure verwendet wird.

| **Lagerstabilität bei 30°C** | | **Start** | **1 Monat** | **3 Monate** | **6 Monate** | **8 Monate** |
|---|---|---|---|---|---|---|
| **Diestergehalt [%]** | min. 85,00 | 88,0 % | 83,5 % | 54,2 % | 49,8 % | 49,5 % |
| **Triestergehalt [%]** | max. 3,00 | 1,7 % | 5,8 % | 29,9 % | 35,5 % | 35,9 % |
| **Trichloressigsäure [%]** | | 0,59 % | n.b. | n.b. | n.b. | n.b. |
| **Glycidylmethacrylat [%]** | | 0,54 % | n.b. | n.b. | n.b. | n.b. |

| **Lagerstabilität bei 50°C** | | **Start** | **1 Monat** | **3 Monate** | **6 Monate** | **8 Monate** |
|---|---|---|---|---|---|---|
| **Diestergehalt [%]** | min. 85,00 | 88,0 % | 50,1 % | 47,9 % | 48,2 % | 48,0 % |
| **Triestergehalt [%]** | max. 3,00 | 1,7 % | 34,1 % | 35,8 % | 36,0 % | 35,8 % |
| **Trichloressigsäure [%]** | | 0,59 % | n.b. | n.b. | n.b. | n.b. |
| **Glycidylmethacrylat [%]** | | 0,54 % | n.b. | n.b. | n.b. | n.b. |

| | | | | | | |
|---|---|---|---|---|---|---|
| Fazit: Das zum Ende der Zugabe der Edukte mit einer molaren Stöchiometrie von 1:1 hergestellte Produkt erwies sich als NICHT lagerstabil! | | | | | | |

### Erfindungsgemäßes Beispiel 39

### Molare Stöchiometrie (Glycidylmethacrylat : Carbonsäure) 1:1,02

Verfahren dadurch gekennzeichnet, dass die Durchführung analog Beispiel 1 durchgeführt wird, jedoch als Carbonsäure 351,7 g Trichloressigsäure verwendet wird.

| **Lagerstabilität bei 30°C** | | **Start** | **1 Monat** | **3 Monate** | **6 Monate** | **8 Monate** |
|---|---|---|---|---|---|---|
| **Diestergehalt [%]** | min. 85,00 | 85,6 % | 86,3 % | 86,1 % | 86,2 % | 85,6 % |
| **Triestergehalt [%]** | max. 3,00 | 1,6 % | 1,6 % | 1,6 % | 1,8 % | 2,0 % |
| **Trichloressigsäure [%]** | | 0,64 % | n.b. | n.b. | n.b. | n.b. |
| **Glycidylmethacrylat [%]** | | 0,48 % | n.b. | n.b. | n.b. | n.b. |

| **Lagerstabilität bei 50°C** | | **Start** | **1 Monat** | **3 Monate** | **6 Monate** | **8 Monate** |
|---|---|---|---|---|---|---|
| **Diestergehalt [%]** | min. 85,00 | 85,6 % | 85,7 % | 85,6 % | 85,1 % | 83,6 % |
| **Triestergehalt [%]** | max. 3,00 | 1,6 % | 1,9 % | 2,2 % | 2,8 % | 3,3 % |
| **Trichloressigsäure [%]** | | 0,64 % | n.b. | n.b. | n.b. | n.b. |
| **Glycidylmethacrylat [%]** | | 0,48 % | n.b. | n.b. | n.b. | n.b. |

| | | | | | | |
|---|---|---|---|---|---|---|
| Fazit: Das zum Ende der Zugabe der Edukte mit einer molaren Stöchiometrie von 1:1,02 hergestellte Produkt hatte eine Lagerstabilität von über 3 Monaten. | | | | | | |

### Erfindungsgemäßes Beispiel 40

### Nachstabilisierung mit systemeigener Carbonsäure vor Lagerung

Verfahren gemäß Beispiel 1, dadurch gekennzeichnet, dass als Carbonsäure 179 g Methacrylsäure verwendet wird und zum Ende der Zugabe der Edukte die molare Stöchiometrie (Glycidylmethacrylat:Carbonsäure) 1:0,985beträgt.

Vor der Lagerung wird das erhaltene Rohprodukt mit 1,5g Methacrylsäure dotiert.

### Molare Stöchiometrie vor Lagerung: Glycidylmethacrylat < Carbonsäure

| **Lagerstabilität bei 30°C** | | **Produk t** | **Start** | **1 Monat** | **3 Monate** | **6 Monate** | **8 Monate** |
|---|---|---|---|---|---|---|---|
| **Diestergehalt [%]** | min. 85,0 0 | 86,10 % | 85,84 % | 86,47 % | 86,28 % | 86,39 % | 85,77 % |
| **Triestergehalt [%]** | max. 3,00 | 1,60 % | 1,59 % | 1,58 % | 1,53 % | 1,69 % | 1,94 % |
| **Methacrylsäure [%]** | | 0,40 % | 0,71 % | n.b. | n.b. | n.b. | n.b. |
| **Glycidylmethacrylat [%]** | | 0,78 % | 0,78 % | n.b. | n.b. | n.b. | n.b. |

| **Lagerstabilität bei 50°C** | | **Produk t** | **Start** | **1 Monat** | **3 Monate** | **6 Monate** | **8 Monate** |
|---|---|---|---|---|---|---|---|
| **Diestergehalt [%]** | min. 85,0 0 | 86,10 % | 85,84 % | 85,94 % | 85,81 % | 85,26 % | 83,81 % |
| **Triestergehalt [%]** | max. 3,00 | 1,60 % | 1,59 % | 1,82 % | 2,10 % | 2,68 % | 3,12 % |
| **Methacrylsäure [%]** | | 0,40 % | 0,71 % | n.b. | n.b. | n.b. | n.b. |
| **Glycidylmethacrylat [%]** | | 0,78 % | 0,78 % | n.b. | n.b. | n.b. | n.b. |

| | | | | | | | |
|---|---|---|---|---|---|---|---|
| Fazit: Das zum Ende der Zugabe der Edukte mit einer molaren Stöchiometrie von 1:0,985 hergestellte, aber nachstabilisierte Rohprodukt hatte eine Lagerstabilität von 6 Monaten, die nicht aufstabilisierte Referenzprobe aus Beispiel 1 war nicht lagerstabil. | | | | | | | |

### Erfindungsgemäßes Beispiel 41

### Nachstabilisierung mit systemeigener Carbonsäure vor Lagerung

Verfahren gemäß Beispiel 2, dadurch gekennzeichnet, dass als Carbonsäure 181,7 g Methacrylsäure verwendet wird und zum Ende der Zugabe der Edukte die molare Stöchiometrie (Glycidylmethacrylat:Carbonsäure) 1:1 betrug.

Vor der Lagerung wird das erhaltene Rohprodukt mit 1,1g Methacrylsäure dotiert.

### Molare Stöchiometrie vor Lagerung: Glycidylmethacrylat < Carbonsäure

| **Lagerstabilität bei 30°C** | | **Produkt** | **Start** | **1 Monat** | **3 Monate** | **6 Monate** | **8 Monate** |
|---|---|---|---|---|---|---|---|
| **Diestergehalt [%]** | min. 85,00 | 86,20 % | 86,00 % | 86,67 % | 86,49 % | 86,59 % | 85,97 % |
| **Triestergehalt [%]** | max. 3,00 | 1,80 % | 1,80 % | 1,67 % | 1,62 % | 1,78 % | 2,05 % |
| **Methacrylsäure [%]** | | 0,42 % | 0,65 % | n.b. | n.b. | n.b. | n.b. |
| **Glycidylmethacrylat [%]** | | 0,72 % | 0,72 % | n.b. | n.b. | n.b. | n.b. |

| **Lagerstabilität bei 50°C** | | **Produkt** | **Start** | **1 Monat** | **3 Monate** | **6 Monate** | **8 Monate** |
|---|---|---|---|---|---|---|---|
| **Diestergehalt [%]** | min. 85,00 | 86,20 % | 86,00 % | 86,14 % | 86,01 % | 85,46 % | 84,00 % |
| **Triestergehalt [%]** | max. 3,00 | 1,80 % | 1,80 % | 1,92 % | 2,22 % | 2,83 % | 3,30 % |
| **Methacrylsäure [%]** | | 0,42 % | 0,65 % | n.b. | n.b. | n.b. | n.b. |
| **Glycidylmethacrylat [%]** | | 0,72 % | 0,72 % | n.b. | n.b. | n.b. | n.b. |

| | | | | | | | |
|---|---|---|---|---|---|---|---|
| Fazit: Das zum Ende der Zugabe der Edukte mit einer molaren Stöchiometrie von 1:1 hergestellte Produkt, aber nachstabilisierte Rohprodukt hatte eine Lagerstabilität von 6 Monaten, die nicht aufstabilisierte Referenzprobe aus Beispiel 2 war nicht lagerstabil. | | | | | | | |

### Erfindungsgemäßes Beispiel 42

### Nachstabilisierung mit einer systemfremden Carbonsäure vor Lagerung

Verfahren gemäß Beispiel 1, dadurch gekennzeichnet, dass als Carbonsäure 179 g Methacrylsäure verwendet wird und zum Ende der Zugabe der Edukte die molare Stöchiometrie (Glycidylmethacrylat:Carbonsäure) 1:0,985 betrug.

Vor der Lagerung wird das erhaltene Rohprodukt mit 0,8 g Ameisensäure dotiert.

### Molare Stöchiometrie vor Lagerung: Glycidylmethacrylat < Carbonsäure

| **Lagerstabilität bei 30°C** | | **Produkt** | **Start** | **1 Monat** | **3 Monate** | **6 Monate** | **8 Monate** |
|---|---|---|---|---|---|---|---|
| **Diestergehalt [%]** | min. 85,0 0 | 86,1 % | 86,0 % | 86,2 % | 86,0 % | 86,1 % | 85,5 % |
| **Triestergehalt [%]** | max. 3,00 | 1,60 % | 1,59 % | 1,18 % | 1,15 % | 1,26 % | 1,45 % |
| **Methacrylsäure [%]** | | 0,40 % | 0,40 % | n.b. | n.b. | n.b. | n.b. |
| **Glycidylmethacrylat [%]** | | 0,78 % | 0,78 % | n.b. | n.b. | n.b. | n.b. |
| **Ameisensäure [%]** | | 0,00 % | 0,16 % | n.b. | n.b. | n.b. | n.b. |

| **Lagerstabilität bei 50°C** | | **Produkt** | **Start** | **1 Monat** | **3 Monate** | **6 Monate** | **8 Monate** |
|---|---|---|---|---|---|---|---|
| **Diestergehalt [%]** | min. 85,0 0 | 86,1 % | 85,8 % | 85,6 % | 85,5 % | 85,0 % | 83,5 % |
| **Triestergehalt [%]** | max. 3,00 | 1,60 % | 1,59 % | 1,36 % | 1,57 % | 2,01 % | 2,34 % |
| **Methacrylsäure [%]** | | 0,40 % | 0,71 % | n.b. | n.b. | n.b. | n.b. |
| **Glycidylmethacrylat [%]** | | 0,78 % | 0,78 % | n.b. | n.b. | n.b. | n.b. |
| **Ameisensäure [%]** | | 0,00 % | 0,16 % | n.b. | n.b. | n.b. | n.b. |

| | | | | | | | |
|---|---|---|---|---|---|---|---|
| Fazit: Das zum Ende der Zugabe der Edukte mit einer molaren Stöchiometrie von 1:0,985 hergestellte, aber mit Ameisensäure als systemfremde Säure nachstabilisierte Rohprodukt hatte eine Lagerstabilität von 6 Monaten, die nicht aufstabilisierte Referenzprobe aus Beispiel 1 war nicht lagerstabil. | | | | | | | |

### Erfindungsgemäßes Beispiel 43

### Nachstabilisierung mit systemfremder Carbonsäure vor Lagerung

Verfahren gemäß Beispiel 2, dadurch gekennzeichnet, dass als Carbonsäure 181,7 g Methacrylsäure verwendet wird und zum Ende der Zugabe der Edukte die molare Stöchiometrie (Glycidylmethacrylat:Carbonsäure) 1:1 betrug.

Vor der Lagerung wird das erhaltene Rohprodukt mit 0,6g Ameisensäure dotiert.

### Molare Stöchiometrie vor Lagerung: Glycidylmethacrylat < Carbonsäure

| **Lagerstabilität bei 30°C** | | **Produkt** | **Start** | **1 Monat** | **3 Monate** | **6 Monate** | **8 Monate** |
|---|---|---|---|---|---|---|---|
| **Diestergehalt [%]** | min. 85,00 | 86,20 % | 86,06 % | 86,67 % | 86,49 % | 86,59 % | 85,97 % |
| **Triestergehalt [%]** | max. 3,00 | 1,80 % | 1,79 % | 1,67 % | 1,62 % | 1,78 % | 2,05 % |
| **Methacrylsäure [%]** | | 0,42 % | 0,65 % | **n.b.** | n.b. | n.b. | n.b. |
| **Glycidylmethacrylat [%]** | | 0,72 % | 0,72 % | **n.b.** | n.b. | n.b. | n.b. |
| **Ameisensäure [%]** | | 0,00 % | 0,12 % | **n.b.** | n.b. | n.b. | n.b. |

| **Lagerstabilität bei 50°C** | | **Produkt** | **Start** | **1 Monat** | **3 Monate** | **6 Monate** | **8 Monate** |
|---|---|---|---|---|---|---|---|
| **Diestergehalt [%]** | min. 85,00 | 86,20 % | 86,00 % | 86,14 % | 86,01 % | 85,46 % | 84,00 % |
| **Triestergehalt [%]** | max. 3,00 | 1,80 % | 1,79 % | 1,92 % | 2,22 % | 2,83 % | 3,30 % |
| **Methacrylsäure [%]** | | 0,42 % | 0,65 % | n.b. | n.b. | n.b. | n.b. |
| **Glycidylmethacrylat [%]** | | 0,72 % | 0,72 % | n.b. | n.b. | n.b. | n.b. |
| **Ameisensäure [%]** | | 0,00 % | 0,12 % | n.b. | n.b. | n.b. | n.b. |

| | | | | | | | |
|---|---|---|---|---|---|---|---|
| Fazit: Das zum Ende der Zugabe der Edukte mit einer molaren Stöchiometrie von 1:1 hergestellte Produkt, aber mit Ameisensäure als systemfremde Säure nachstabilisierte Rohprodukt hatte eine Lagerstabilität von 6 Monaten, die nicht aufstabilisierte Referenzprobe aus Beispiel 2 war nicht lagerstabil. | | | | | | | |

### Erfindungsgemäßes Beispiel 44

### Nachstabilisierung mit einer Broenstedsäure vor Lagerung

Verfahren gemäß Beispiel 1, dadurch gekennzeichnet, dass als Carbonsäure 179 g Methacrylsäure verwendet wird und zum Ende der Zugabe der Edukte die Molare Stöchiometrie (Glycidylmethacrylat:Carbonsäure) 1:0,985 betrug. Vor der Lagerung wird das erhaltene Rohprodukt mit 0,55g Phosphorsäure dotiert.

### Molare Stöchiometrie vor Lagerung: Glycidylmethacrylat < Säure

| **Lagerstabilität bei 30°C** | | **Produkt** | **Start** | **1 Monat** | **3 Monate** | **6 Monate** | **8 Monate** |
|---|---|---|---|---|---|---|---|
| **Diestergehalt [%]** | min. 85,00 | 86,1 % | 86,0 % | 86,2 % | 86,0 % | 86,1 % | 85,5 % |
| **Triestergehalt [%]** | max. 3,00 | 1,60 % | 1,59 % | 1,18 % | 1,15 % | 1,26 % | 1,45 % |
| **Methacrylsäure [%]** | | 0,40 % | 0,40 % | n.b. | n.b. | n.b. | n.b. |
| **Glycidylmethacrylat [%]** | | 0,78 % | 0,78 % | n.b. | n.b. | n.b. | n.b. |
| **Phosphorsäure [%]** | | 0,00 % | 0,11 % | n.b. | n.b. | n.b. | n.b. |

| **Lagerstabilität bei 50°C** | | **Produkt** | **Start** | **1 Monat** | **3 Monate** | **6 Monate** | **8 Monate** |
|---|---|---|---|---|---|---|---|
| **Diestergehalt [%]** | min. 85,00 | 86,1 % | 86,0 % | 85,6 % | 85,5 % | 85,0 % | 83,5 % |
| **Triestergehalt [%]** | max. 3,00 | 1,60 % | 1,59 % | 1,36 % | 1,57 % | 2,01 % | 2,34 % |
| **Methacrylsäure [%]** | | 0,40 % | 0,71 % | n.b. | n.b. | n.b. | n.b. |
| **Glycidylmethacrylat [%]** | | 0,78 % | 0,78 % | n.b. | n.b. | n.b. | n.b. |
| **Phosphorsäure [%]** | | 0,00 % | 0,11 % | n.b. | n.b. | n.b. | n.b. |

| | | | | | | | |
|---|---|---|---|---|---|---|---|
| Fazit: Das zum Ende der Zugabe der Edukte mit einer molaren Stöchiometrie von 1:0,985 hergestellte, aber mit Phosphorsäure als anorganische, systemfremde Säure nachstabilisierte Rohprodukt hatte eine Lagerstabilität von 6 Monaten, die nicht aufstabilisierte Referenzprobe aus Beispiel 1 war nicht lagerstabil. | | | | | | | |

### Erfindungsgemäßes Beispiel 45

Nachstabilisierung mit einer Broenstedsäure vor Lagerung Verfahren gemäß Beispiel 2, dadurch gekennzeichnet, dass als Carbonsäure 181,7 g Methacrylsäure verwendet wird und zum Ende der Zugabe der Edukte die Molare Stöchiometrie (Glycidylmethacrylat:Carbonsäure) 1:1 betrug. Vor der Lagerung wird das erhaltene Rohprodukt mit 0,25 g Phosphorsäure dotiert.

### Molare Stöchiometrie vor Lagerung: Glycidylmethacrylat < Säure

| **Lagerstabilität bei 30°C** | | **Produkt** | **Start** | **1 Monat** | **3 Monate** | **6 Monate** | **8 Monate** |
|---|---|---|---|---|---|---|---|
| **Diestergehalt [%]** | min. 85,00 | 86,20 % | 86,06 % | 86,67 % | 86,49 % | 86,59 % | 85,97 % |
| **Triestergehalt [%]** | max. 3,00 | 1,80 % | 1,79 % | 1,67 % | 1,62 % | 1,78 % | 2,05 % |
| **Methacrylsäure [%]** | | 0,42 % | 0,65 % | n.b. | n.b. | n.b. | n.b. |
| **Glycidylmethacrylat [%]** | | 0,72 % | 0,72 % | n.b. | n.b. | n.b. | n.b. |
| **Phosphorsäure [%]** | | 0,00 % | 0,05 % | n.b. | n.b. | n.b. | n.b. |

| **Lagerstabilität bei 50°C** | | **Produkt** | **Start** | **1 Monat** | **3 Monate** | **6 Monate** | **8 Monate** |
|---|---|---|---|---|---|---|---|
| **Diestergehalt [%]** | min. 85,00 | 86,20 % | 86,06 % | 86,14 % | 86,01 % | 85,46 % | 84,00 % |
| **Triestergehalt [%]** | max. 3,00 | 1,80 % | 1,79 % | 1,92 % | 2,22 % | 2,83 % | 3,30 % |
| **Methacrylsäure [%]** | | 0,42 % | 0,65 % | n.b. | n.b. | n.b. | n.b. |
| **Glycidylmethacrylat [%]** | | 0,72 % | 0,72 % | n.b. | n.b. | n.b. | n.b. |
| **Phosphorsäure [%]** | | 0,00 % | 0,05 % | n.b. | n.b. | n.b. | n.b. |

| | | | | | | | |
|---|---|---|---|---|---|---|---|
| Fazit: Das zum Ende der Zugabe der Edukte mit einer molaren Stöchiometrie von 1:1 hergestellte Produkt, aber mit Phosphorsäure als anorganische, systemfremde Säure nachstabilisierte Rohprodukt hatte eine Lagerstabilität von 6 Monaten, die nicht aufstabilisierte Referenzprobe aus Beispiel 2 war nicht lagerstabil. | | | | | | | |

## Patentansprüche

1. Lagerstabile Glycerin(meth)acrylatcarbonsäureester der Formel (I) 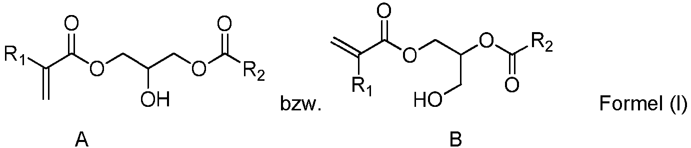 mit
R₁ = H oder CH₃
R₂=Wasserstoff, oder aliphatische Kohlenstoffe mit C1 bis C30, oder aliphatisch cyclische Kohlenstoffverbindungen mit einer Ringgröße von C4 bis C8, unsubstituiert oder substituiert, mit N, S, O oder P, oder halogenierte aliphatische Kohlenstoffverbindungen mit C1 bis C8, oder aromatische Kohlenstoffverbindungen, oder heteroaromatische Kohlenstoffverbindungen mit N, S, O oder P substituiert, oder
ungesättigten aliphatischen Kohlenstoffverbindungen mit C2 bis C30,
**dadurch gekennzeichnet, dass** systemeigene Carbonsäuren der Formel (II) 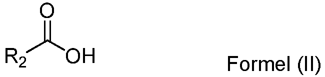 wobei
R₂=Wasserstoff, oder aliphatische Kohlenstoffe mit C1 bis C30, oder aliphatisch cyclische Kohlenstoffverbindungen mit einer Ringgröße von C4 bis C8, unsubstituiert oder substituiert, mit N, S, O oder P, oder halogenierte aliphatische Kohlenstoffverbindungen mit C1 bis C8, oder aromatische Kohlenstoffverbindungen, oder heteroaromatische Kohlenstoffverbindungen mit N, S, O oder P substituiert, oder
ungesättigten aliphatischen Kohlenstoffverbindungen mit C2 bis C30
und systemfremde Carbonsäuren der Formel (III) 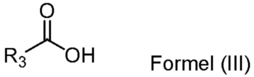 wobei
R₃=Wasserstoff, oder aliphatische Kohlenstoffe mit C1 bis C30, oder aliphatisch cyclische Kohlenstoffverbindungen mit einer Ringgröße von C4 bis C8, unsubstituiert oder substituiert, mit N, S, O oder P, oder halogenierte aliphatische Kohlenstoffverbindungen mit C1 bis C8, oder aromatische Kohlenstoffverbindungen, oder heteroaromatische Kohlenstoffverbindungen mit N, S, O oder P substituiert, oder
ungesättigten aliphatischen Kohlenstoffverbindungen mit C2 bis C30 einzeln oder in Mischungen in Summe im molaren Überschuss zum enthaltenden Glycidyl(meth)acrylat enthalten sind.

2. Lagerstabile Glycerin(meth)acrylatcarbonsäureester gemäß Anspruch 1, **dadurch gekennzeichnet, dass** systemeigene Carbonsäuren der Formel (II), systemfremde Carbonsäuren der Formel (III) und beliebige weitere systemfremde Broenstedsäuren in Summe im molaren Überschuss zum enthaltenden Glycidyl(meth)acrylat enthalten sind.

3. Lagerstabile Glycerin(meth)acrylatcarbonsäureester gemäß Anspruch 1, **dadurch gekennzeichnet, dass** der Überschuss von beliebigen Mischungen der Carbonsäuren gemäß Formel (II) und (III) im molaren Verhältnis von 1,001 zu 1 bis 5 zu 1 zum Ende der Zugabe der Edukte enthalten sind.

4. Lagerstabile Glycerin(meth)acrylatcarbonsäureester gemäß Anspruch 1, **dadurch gekennzeichnet, dass** ein Überschuss von beliebigen Mischungen der Carbonsäuren gemäß Formel (II) und (III) im molaren Verhältnis von 1,01 zu 1 bis 2 zu 1, bevorzugt 1,02 zu 1 bis 1,5 zu 1, zum Ende der Zugabe der Edukte enthalten sind.

5. Lagerstabile Glycerin(meth)acrylatcarbonsäureester der Formel (I) 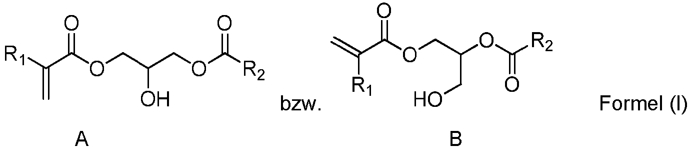 mit
R₁ = H oder CH₃
R₂=Wasserstoff, oder aliphatische Kohlenstoffe mit C1 bis C30, oder aliphatisch cyclische Kohlenstoffverbindungen mit einer Ringgröße von C4 bis C8, unsubstituiert oder substituiert, mit N, S, O oder P, oder halogenierte aliphatische Kohlenstoffverbindungen mit C1 bis C8, oder aromatische Kohlenstoffverbindungen, oder heteroaromatische Kohlenstoffverbindungen mit N, S, O oder P substituiert, oder
ungesättigten aliphatischen Kohlenstoffverbindungen mit C2 bis C30,
**dadurch gekennzeichnet, dass**
Carbonsäuren der Formel (II) 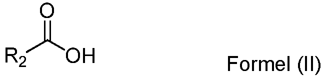 wobei
R₂=Wasserstoff, oder aliphatische Kohlenstoffe mit C1 bis C30, oder aliphatisch cyclische Kohlenstoffverbindungen mit einer Ringgröße von C4 bis C8, unsubstituiert oder substituiert, mit N, S, O oder P, oder halogenierte aliphatische Kohlenstoffverbindungen mit C1 bis C8, oder aromatische Kohlenstoffverbindungen, oder heteroaromatische Kohlenstoffverbindungen mit N, S, O oder P substituiert, oder
ungesättigten aliphatischen Kohlenstoffverbindungen mit C2 bis C30
und gegebenenfalls Carbonsäuren der Formel (III) 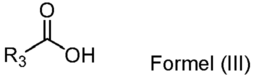 wobei
R₃=Wasserstoff, oder aliphatische Kohlenstoffe mit C1 bis C30, oder aliphatisch cyclische Kohlenstoffverbindungen mit einer Ringgröße von C4 bis C8, unsubstituiert oder substituiert, mit N, S, O oder P, oder halogenierte aliphatische Kohlenstoffverbindungen mit C1 bis C8, oder aromatische Kohlenstoffverbindungen, oder heteroaromatische Kohlenstoffverbindungen mit N, S, O oder P substituiert, oder
ungesättigten aliphatischen Kohlenstoffverbindungen mit C2 bis C30 einzeln oder in Mischungen
und gegebenenfalls
beliebige weitere systemfremde Broenstedsäuren in Summe im molaren Überschuss zum enthaltenden Glycidyl(meth)acrylat zu Beginn der Lagerung enthalten sind.

6. Lagerstabile Glycerin(meth)acrylatcarbonsäureester gemäß Anspruch 5, **dadurch gekennzeichnet, dass** ein Überschuss von beliebigen Mischungen der Carbonsäuren gemäß Formel (III) im molaren Verhältnis von 1,001 zu 1 bis 5 zu 1 zum verbleibenden Glycidyl(meth)acrylat zu Beginn der Lagerung enthalten sind.

7. Lagerstabile Glycerin(meth)acrylatcarbonsäureester gemäß Anspruch 5, **dadurch gekennzeichnet, dass** ein Überschuss von beliebigen Mischungen der Carbonsäuren gemäß Formel (III) im molaren Verhältnis von 1,01 zu 1 bis 2 zu 1, bevorzugt von 1,02 zu 1 bis 1,5 zu 1, zum verbleibenden Glycidyl(meth)acrylat zu Beginn der Lagerung enthalten sind.

8. Lagerstabile Glycerin(meth)acrylatcarbonsäureester gemäß der Ansprüche 1 oder 5, **dadurch gekennzeichnet, dass** eine beliebige Säure und Glycidyl(meth)acrylat im molaren Verhältnis von 1,001 zu 1 bis 5 zu 1 im Produkt zu Beginn der Lagerung enthalten sind.

9. Verfahren zur Herstellung von lagerstabilem Glycerin(meth)acrylatcarbonsäureester nach Anspruch 1 oder 5, **dadurch gekennzeichnet, dass** systemeigene Carbonsäuren der Formel (II) 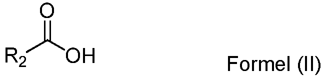 wobei
R₂=Wasserstoff, oder aliphatische Kohlenstoffe mit C1 bis C30, oder aliphatisch cyclische Kohlenstoffverbindungen mit einer Ringgröße von C4 bis C8, unsubstituiert oder substituiert, mit N, S, O oder P, oder halogenierte aliphatische Kohlenstoffverbindungen mit C1 bis C8, oder aromatische Kohlenstoffverbindungen, oder heteroaromatische Kohlenstoffverbindungen mit N, S, O oder P substituiert, oder
ungesättigten aliphatischen Kohlenstoffverbindungen mit C2 bis C30 und Glycidyl(meth)acrylat in Anwesenheit eines Katalysators bei Temperaturen von 20-130°C umgesetzt werden, indem systemeigenen Carbonsäure (II) vorgelegt und Glycidyl(meth)acrylat kontinuierlich zugegeben wird, und systemfremde Carbonsäuren der Formel (III) 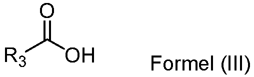 wobei
R₃=Wasserstoff, oder aliphatische Kohlenstoffe mit C1 bis C30, oder aliphatisch cyclische Kohlenstoffverbindungen mit einer Ringgröße von C4 bis C8, unsubstituiert oder substituiert, mit N, S, O oder P, oder halogenierte aliphatische Kohlenstoffverbindungen mit C1 bis C8, oder aromatische Kohlenstoffverbindungen, oder heteroaromatische Kohlenstoffverbindungen mit N, S, O oder P substituiert, oder
ungesättigten aliphatischen Kohlenstoffverbindungen mit C2 bis C30 einzeln oder in Mischungen
in Summe im molaren Überschuss zum enthaltenden Glycidyl(meth)acrylat zugegeben werden.

10. Verfahren nach Anspruch 9, **dadurch gekennzeichnet, dass** das oder vor der Lagerung systemeigene Carbonsäuren (II), systemfremde Carbonsäuren (III) und/oder Broenstedsäuren zugegeben werden.

11. Verfahren nach Anspruch 9, **dadurch gekennzeichnet, dass** das molare Verhältnis Carbonsäure (II) und (III) zu Glycidyl(meth)acrylat am Ende der Eduktzugabe auf 1,001 zu 1 bis 5 zu 1 durch Vorlage der Carbonsäuren (II) und (III) eingestellt wird.

12. Verfahren nach Anspruch 9, **dadurch gekennzeichnet, dass** das molare Verhältnis Carbonsäure (III) zu Glycidyl(meth)acrylat am Ende der Eduktzugabe auf 1,001 zu 1 bis 5 zu 1 durch Vorlage der Carbonsäure (III) eingestellt wird.

13. Verfahren nach Anspruch 9, **dadurch gekennzeichnet, dass** das molare Verhältnis Säure zu Glycidyl(meth)acrylat zu Beginn der Lagerung auf 1,001 zu 1 bis 5 zu 1 durch Zugabe einer Säure eingestellt wird.

14. Verfahren nach Anspruch 9, **dadurch gekennzeichnet, dass** Glycidyl(meth)acrylat und Carbonsäure (II) in Anwesenheit katalytischer Mengen eines quaternären Alkylammoniumhalogenids umgesetzt werden.

15. Verfahren nach Anspruch 13, **dadurch gekennzeichnet, dass** das quaternäre Alkylammoniumhalogenid Benzyltriethylammoniumchlorid ist.

16. Lagerstabile Glycerin(meth)acrylatcarbonsäureester gemäß der Ansprüche 1 oder 5, **dadurch gekennzeichnet, dass** die Carbonsäure (Meth)acrylsäure ist.

## Claims

1. Storage-stable glycerol (meth)acrylate carboxylic esters of formula (I) 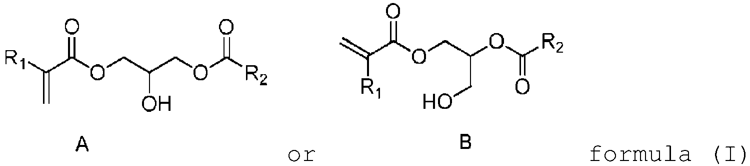 where
R₁ = H or CH₃,
R₂ = hydrogen, or aliphatic carbons with C1 to C30, or aliphatic cyclic carbon compounds with a ring size of C4 to C8, unsubstituted or substituted by N, S, O or P, or halogenated aliphatic carbon compounds with C1 to C8, or aromatic carbon compounds, or heteroaromatic carbon compounds substituted by N, S, O or P, or
unsaturated aliphatic carbon compounds with C2 to C30, **characterized in that** carboxylic acids of formula (II) endogenous to the system 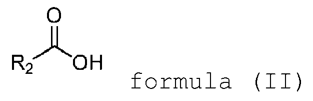 where
R₂ = hydrogen, or aliphatic carbons with C1 to C30, or aliphatic cyclic carbon compounds with a ring size of C4 to C8, unsubstituted or substituted by N, S, O or P, or halogenated aliphatic carbon compounds with C1 to C8, or aromatic carbon compounds, or heteroaromatic carbon compounds substituted by N, S, O or P, or
unsaturated aliphatic carbon compounds with C2 to C30 and carboxylic acids of formula (III) extraneous to the system 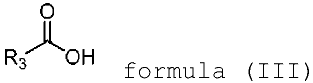 where
R₃ = hydrogen, or aliphatic carbons with C1 to C30, or aliphatic cyclic carbon compounds with a ring size of C4 to C8, unsubstituted or substituted by N, S, O or P, or halogenated aliphatic carbon compounds with C1 to C8, or aromatic carbon compounds, or heteroaromatic carbon compounds substituted by N, S, O or P, or
unsaturated aliphatic carbon compounds with C2 to C30 individually or in mixtures are present in sum total in a molar excess to the glycidyl (meth)acrylate present.

2. Storage-stable glycerol (meth)acrylate carboxylic esters according to Claim 1, **characterized in that** carboxylic acids of formula (II) endogenous to the system, carboxylic acids of formula (III) extraneous to the system and any further Brønsted acids extraneous to the system are present in sum total in a molar excess to the glycidyl (meth)acrylate present.

3. Storage-stable glycerol (meth)acrylate carboxylic esters according to Claim 1, **characterized in that** the excess of any mixtures of the carboxylic acids according to formula (II) and (III) is present in a molar ratio of 1.001:1 to 5:1 at the end of the addition of the reactants.

4. Storage-stable glycerol (meth)acrylate carboxylic esters according to Claim 1, **characterized in that** an excess of any mixtures of the carboxylic acids according to formula (II) and (III) is present in a molar ratio of 1.01:1 to 2:1, preferably of 1.02:1 to 1.5:1, at the end of the addition of the reactants.

5. Storage-stable glycerol (meth)acrylate carboxylic esters of formula (I) 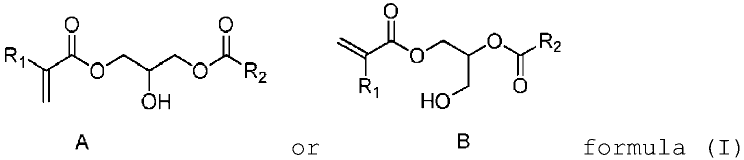 where
R₁ = H or CH₃,
R₂ = hydrogen, or aliphatic carbons with C1 to C30, or aliphatic cyclic carbon compounds with a ring size of C4 to C8, unsubstituted or substituted by N, S, O or P, or halogenated aliphatic carbon compounds with C1 to C8, or aromatic carbon compounds, or heteroaromatic carbon compounds substituted by N, S, O or P, or
unsaturated aliphatic carbon compounds with C2 to C30, **characterized in that**
carboxylic acids of formula (II) 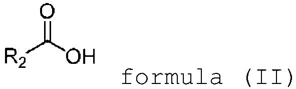 where
R₂ = hydrogen, or aliphatic carbons with C1 to C30, or aliphatic cyclic carbon compounds with a ring size of C4 to C8, unsubstituted or substituted by N, S, O or P, or halogenated aliphatic carbon compounds with C1 to C8, or aromatic carbon compounds, or heteroaromatic carbon compounds substituted by N, S, O or P, or
unsaturated aliphatic carbon compounds with C2 to C30 and optionally carboxylic acids of formula (III) 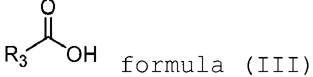 where
R₃ = hydrogen, or aliphatic carbons with C1 to C30, or aliphatic cyclic carbon compounds with a ring size of C4 to C8, unsubstituted or substituted by N, S, O or P, or halogenated aliphatic carbon compounds with C1 to C8, or aromatic carbon compounds, or heteroaromatic carbon compounds substituted by N, S, O or P, or
unsaturated aliphatic carbon compounds with C2 to C30 individually or in mixtures
and optionally
any further Brønsted acids extraneous to the system are present at the beginning of the storage in sum total in a molar excess to the glycidyl (meth)acrylate present.

6. Storage-stable glycerol (meth)acrylate carboxylic esters according to Claim 5, **characterized in that** an excess of any mixtures of the carboxylic acids according to formula (III) is present at the beginning of the storage in a molar ratio to the remaining glycidyl (meth)acrylate of 1.001:1 to 5:1.

7. Storage-stable glycerol (meth)acrylate carboxylic esters according to Claim 5, **characterized in that** an excess of any mixtures of the carboxylic acids according to formula (III) is present at the beginning of the storage in a molar ratio to the remaining glycidyl (meth)acrylate of 1.01:1 to 2:1, preferably of 1.02:1 to 1.5:1.

8. Storage-stable glycerol (meth)acrylate carboxylic esters according to Claims 1 or 5, **characterized in that** any desired acid and glycidyl (meth)acrylate are present in the product at the beginning of the storage in a molar ratio of 1.001:1 to 5:1.

9. Process for preparing storage-stable glycerol (meth)acrylate carboxylic ester according to Claim 1 or 5, **characterized in that** carboxylic acids of formula (II) endogenous to the system 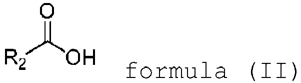 where
R₂ = hydrogen, or aliphatic carbons with C1 to C30, or aliphatic cyclic carbon compounds with a ring size of C4 to C8, unsubstituted or substituted by N, S, O or P, or halogenated aliphatic carbon compounds with C1 to C8, or aromatic carbon compounds, or heteroaromatic carbon compounds substituted by N, S, O or P, or
unsaturated aliphatic carbon compounds with C2 to C30
and glycidyl (meth)acrylate are reacted at temperatures of 20-130°C in the presence of a catalyst, by initially charging carboxylic acid (II) endogenous to the system and adding glycidyl (meth)acrylate continuously, and adding carboxylic acids of formula (III) extraneous to the system 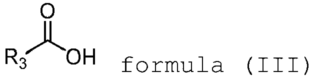 where
R₃ = hydrogen, or aliphatic carbons with C1 to C30, or aliphatic cyclic carbon compounds with a ring size of C4 to C8, unsubstituted or substituted by N, S, O or P, or halogenated aliphatic carbon compounds with C1 to C8, or aromatic carbon compounds, or heteroaromatic carbon compounds substituted by N, S, O or P, or
unsaturated aliphatic carbon compounds with C2 to C30 individually or in mixtures
in sum total in a molar excess to the glycidyl (meth)acrylate present.

10. Process according to Claim 9, **characterized in that** the or prior to the storage carboxylic acids (II) endogenous to the system, carboxylic acids (III) extraneous to the system and/or Brønsted acids are added.

11. Process according to Claim 9, **characterized in that** the molar ratio of carboxylic acid (II) and (III) to glycidyl (meth)acrylate at the end of the reactant addition is adjusted to 1.001:1 to 5:1 by addition of the carboxylic acids (II) and (III).

12. Process according to Claim 9, **characterized in that** the molar ratio of carboxylic acid (III) to glycidyl (meth)acrylate at the end of the reactant addition is adjusted to 1.001:1 to 5:1 by addition of the carboxylic acid (III).

13. Process according to Claim 9, **characterized in that** the molar ratio of acid to glycidyl (meth)acrylate at the beginning of the storage is adjusted to 1.001:1 to 5:1 by addition of an acid.

14. Process according to Claim 9, **characterized in that** glycidyl (meth)acrylate and carboxylic acid (II) are reacted in the presence of catalytic amounts of a quaternary alkylammonium halide.

15. Process according to Claim 13, **characterized in that** the quaternary alkylammonium halide is benzyltriethylammonium chloride.

16. Storage-stable glycerol (meth)acrylate carboxylic esters according to Claims 1 or 5, **characterized in that** the carboxylic acid is (meth)acrylic acid.

## Revendications

1. Esters carboxyliques de (méth)acrylate de glycérol stables au stockage de formule (I) 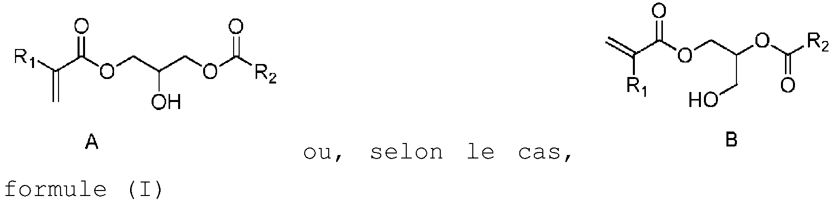 dans laquelle
R₁ = H ou CH₃
R₂ = hydrogène ou carbones aliphatiques en C1 à C30, ou composés carbonés aliphatiquement cycliques d'une dimension de cycle de C4 à C8, non substitués ou substitués par N, S, O ou P, ou composés carbonés aliphatiques halogénés en C1 à C8, ou composés carbonés aromatiques ou composés carbonés hétéroaromatiques substitués par N, S, O ou P ou
composés carbonés aliphatiques insaturés en C2 à C30,
**caractérisés en ce que** des acides carboxyliques de formule (II) propres au système 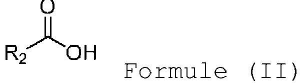 dans laquelle
R₂ = hydrogène ou carbones aliphatiques en C1 à C30, ou composés carbonés aliphatiquement cycliques d'une dimension de cycle de C4 à C8, non substitués ou substitués par N, S, O ou P, ou composés carbonés aliphatiques halogénés en C1 à C8, ou composés carbonés aromatiques ou composés carbonés hétéroaromatiques substitués par N, S, O ou P ou
composés carbonés aliphatiques insaturés en C2 à C30
et des acides carboxyliques de formule (III) étrangers au système 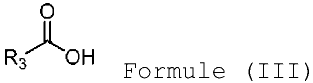 dans laquelle
R₃ = hydrogène ou carbones aliphatiques en C1 à C30, ou composés carbonés aliphatiquement cycliques d'une dimension de cycle de C4 à C8, non substitués ou substitués par N, S, O ou P, ou composés carbonés aliphatiques halogénés en C1 à C8, ou composés carbonés aromatiques ou composés carbonés hétéroaromatiques substitués par N, S, O ou P ou
composés carbonés aliphatiques insaturés en C2 à C30, sont contenus seuls ou dans des mélanges au total en un excès molaire par rapport au (méth)acrylate de glycidyle contenu.

2. Esters carboxyliques de (méth)acrylate de glycérol stables au stockage selon la revendication 1, **caractérisés en ce que** les acides carboxyliques de formule (II) propres au système, les acides carboxyliques de formule (III) étrangers au système et d'autres acides de Bronsted étrangers au système sont contenus au total en un excès molaire par rapport au (méth)acrylate de glycidyle contenu.

3. Esters carboxyliques de (méth)acrylate de glycérol stables au stockage selon la revendication 1, **caractérisés en ce que** l'excès de mélanges quelconques des acides carboxyliques selon la formule (II) et (III) est contenu dans un rapport molaire de 1,001:1 à 5:1 à la fin de l'ajout des produits de départ.

4. Esters carboxyliques de (méth)acrylate de glycérol stables au stockage selon la revendication 1, **caractérisés en ce qu'**un excès de mélanges quelconques des acides carboxyliques selon la formule (II) et (III) est contenu dans un rapport molaire de 1,01:1 à 2:1, de préférence de 1,02:1 à 1,5:1, à la fin de l'ajout des produits de départ.

5. Esters carboxyliques de (méth)acrylate de glycérol stables au stockage de formule (I) 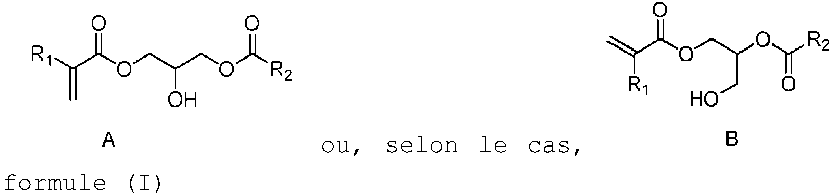 dans laquelle
R₁ = H ou CH₃
R₂ = hydrogène ou carbones aliphatiques en C1 à C30, ou composés carbonés aliphatiquement cycliques d'une dimension de cycle de C4 à C8, non substitués ou substitués par N, S, O ou P, ou composés carbonés aliphatiques halogénés en C1 à C8, ou composés carbonés aromatiques ou composés carbonés hétéroaromatiques substitués par N, S, O ou P ou
composés carbonés aliphatiques insaturés en C2 à C30,
**caractérisés en ce que**
des acides carboxyliques de formule (II) 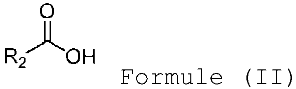 dans laquelle
R₂ = hydrogène ou carbones aliphatiques en C1 à C30, ou composés carbonés aliphatiquement cycliques d'une dimension de cycle de C4 à C8, non substitués ou substitués par N, S, O ou P, ou composés carbonés aliphatiques halogénés en C1 à C8, ou composés carbonés aromatiques ou composés carbonés hétéroaromatiques substitués par N, S, O ou P ou
composés carbonés aliphatiques insaturés en C2 à C30
et le cas échéant des acides carboxyliques de formule (III) 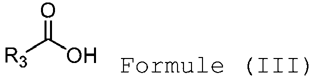 dans laquelle
R₃ = hydrogène ou carbones aliphatiques en C1 à C30, ou composés carbonés aliphatiquement cycliques d'une dimension de cycle de C4 à C8, non substitués ou substitués par N, S, O ou P, ou composés carbonés aliphatiques halogénés en C1 à C8, ou composés carbonés aromatiques ou composés carbonés hétéroaromatiques substitués par N, S, O ou P ou
composés carbonés aliphatiques insaturés en C2 à C30, seuls ou en mélanges
et le cas échéant
de quelconques autres acides de Bronsted étrangers au système sont contenus au total en excès molaire par rapport au (méth)acrylate de glycidyle contenu au début du stockage.

6. Esters carboxyliques de (méth)acrylate de glycérol stables au stockage selon la revendication 5, **caractérisés en ce qu'**un excès de mélanges quelconques des acides carboxyliques selon la formule (III) est contenu dans un rapport molaire de 1,001:1 à 5:1 par rapport au (méth)acrylate de glycidyle contenu au début du stockage.

7. Esters carboxyliques de (méth)acrylate de glycérol stables au stockage selon la revendication 5, **caractérisés en ce qu'**un excès de mélanges quelconques des acides carboxyliques selon la formule (III) est contenu dans un rapport molaire de 1,01:1 à 2:1, de préférence de 1,02:1 à 1,5:1, par rapport au (méth)acrylate de glycidyle contenu au début du stockage.

8. Esters carboxyliques de (méth)acrylate de glycérol stables au stockage selon la revendication 1 ou 5, **caractérisés en ce qu'**un acide quelconque et le (méth)acrylate de glycidyle sont contenus dans un rapport molaire de 1,001:1 à 5:1 dans le produit au début du stockage.

9. Procédé de préparation d'esters carboxyliques de (méth)acrylate de glycérol stables au stockage selon la revendication 1 ou 5, **caractérisé en ce que** des acides carboxyliques de formule (II) propres au système 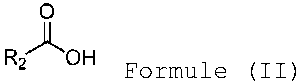 dans laquelle
R₂ = hydrogène ou carbones aliphatiques en C1 à C30, ou composés carbonés aliphatiquement cycliques d'une dimension de cycle de C4 à C8, non substitués ou substitués par N, S, O ou P, ou composés carbonés aliphatiques halogénés en C1 à C8, ou composés carbonés aromatiques ou composés carbonés hétéroaromatiques substitués par N, S, O ou P ou
composés carbonés aliphatiques insaturés en C2 à C30
et du (méth)acrylate de glycidyle sont transformés en présence d'un catalyseur à des températures de 20-130 °C **en ce qu'**un acide carboxylique (II) propre au système est disposé au préalable et du (méth)acrylate de glycidyle est ajouté en continu, et des acides carboxyliques de formule (III) étrangers au système 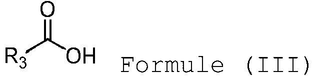 dans laquelle
R₃ = hydrogène ou carbones aliphatiques en C1 à C30, ou composés carbonés aliphatiquement cycliques d'une dimension de cycle de C4 à C8, non substitués ou substitués par N, S, O ou P, ou composés carbonés aliphatiques halogénés en C1 à C8, ou composés carbonés aromatiques ou composés carbonés hétéroaromatiques substitués par N, S, O ou P ou
composés carbonés aliphatiques insaturés en C2 à C30, seuls ou en mélanges
sont ajoutés au total en excès molaire par rapport au (méth)acrylate de glycidyle contenu.

10. Procédé selon la revendication 9, **caractérisé en ce que** le ou avant le stockage des acides carboxyliques (II) propres au système, des acides carboxyliques (III) étrangers au système et/ou des acides de Bronsted sont ajoutés.

11. Procédé selon la revendication 9, **caractérisé en ce que** le rapport molaire acide carboxylique (II) et (III) à (méth)acrylate de glycidyle à la fin de l'ajout des produits de départ est réglé à 1,001:1 à 5:1 par disposition au préalable des acides carboxyliques (II) et (III).

12. Procédé selon la revendication 9, **caractérisé en ce que** le rapport molaire acide carboxylique (III) à (méth)acrylate de glycidyle à la fin de l'ajout des produits de départ est réglé à 1,001:1 à 5:1 par disposition au préalable de l'acide carboxylique (III).

13. Procédé selon la revendication 9, **caractérisé en ce que** le rapport molaire acide à (méth)acrylate de glycidyle au début du stockage est réglé à 1,001:1 à 5:1 par ajout d'un acide.

14. Procédé selon la revendication 9, **caractérisé en ce que** le (méth)acrylate de glycidyle et l'acide carboxylique (II) sont transformés en présence de quantités catalytiques d'un halogénure d'alkylammonium quaternaire.

15. Procédé selon la revendication 13, **caractérisé en ce que** l'halogénure d'alkylammonium quaternaire est le chlorure de benzyltriéthylammonium.

16. Esters carboxyliques de (méth)acrylate de glycérol stables au stockage selon la revendication 1 ou 5, **caractérisés en ce que** l'acide carboxylique est l'acide (méth) acrylique.
